# EUROPEAN PATENT SPECIFICATION

(11) **EP 1 651 196 B1**
(45) Date of publication and mention of the grant of the patent: **25.02.2009**
(21) Application number: 04760495.4
(22) Date of filing: 23.03.2004
(51) Int. Cl.: A61K 9/70, A61K 31/4468, A61K 31/485, A61K 9/00

(54) **An abuse-resistant transdermal dosage form comprising an active agent and an antagonist to said active agent**
Eine Missbrauch verhinderende, transdermale Dosierungsform, die einen Wirkstoff und einen Antagonisten des Wirkstoffs enthält
Une forme posologique anti-abus transdermique contenant un principe actif et un antagoniste de ce principe actif

(30) Priority: 30.04.2003 US 467243 P; 23.12.2003 US 744966
(43) Date of publication of application: 03.05.2006
(73) Proprietor: 3M Innovative Properties Company, St. Paul MN 55133-3427 (US)
(72) Inventor: Hart, John R., c/o 3M Center, Saint Paul, MN 55133-3427 (US); Anderson, Steven C., c/o 3M Center, Saint Paul, MN 55133-3427 (US); Vanblaricom, Melissa A., c/o 3M Center, Saint Paul, MN 55133-3427 (US); Bosl, Ellen R., c/o 3M Center, Saint Paul, MN 55133-3427 (US)
(74) Representative: Vossius & Partner
(86) International application number: PCT/US2004/008856
(87) International publication number: WO 2004/098568

(56) References cited:
- WO-A-97/04835
- WO-A-02/087482
- WO-A-03/090729
- US-A- 5 066 494
- US-A- 5 149 538

## Description

The present invention relates to transdermal dosage forms which are useful for preventing or discouraging tampering, abuse, misuse or diversion of a dosage form containing an active pharmaceutical agent, such as an opioid. The present invention also relates to methods of treating a patient with such a dosage form.

Transdermal drug delivery is a well known method for administering pharmaceuticals. The potential for abuse or misuse of certain pharmaceuticals, such as narcotics and other psychoactive drugs, is also well known. It is thus desirable to prevent or discourage tampering, abuse, misuse or diversion of a transdermal dosage form containing a substance with the potential for abuse or misuse. Although a transdermal dosage form is intended to deliver drug across the skin, abuse of such a dosage can take place by other modes, including oral, buccal, and intravenous.

Methods of combining an abusable substance with an antagonist for the abusable substance have been previously proposed. U.S. Patent No. 5,236,714 (Lee et al.) describes a composition of matter adapted for topical administration to the skin or mucosa which comprises a mixture of an abusable substance in combination with an amount of antagonist for the abusable substance sufficient to substantially negate the pharmacological effect of the abusable substance. U.S. Patent No. 5,149,538 (Granger et al.) describes a misuse-resistive dosage form comprising an opioid permeable to the skin, an antagonist for the opioid releasable upon ingestion or solvent immersion, and an impermeable barrier means separating the opioid and the antagonist.

US-A-5,066,494 describes a transdermal therapeutic system for administering or applying active substances to the skin with a backing layer remote from the skin, an active substance depot, an active substance delivery control means controlling the delivery of the active substance through the system and a contact adhesive means for fixing the therapeutic system to the skin, in which the active substance depot is a multichamber system, in which discreet chambers contain one or more active substances.

US-A-5,149,538 relates to a misuse-resistive dosage form for the transdermal delivery of opioid which comprises, in combination, 1) one or more opioids permeable to the skin, 2) delivery means permeable to the opioid, 3) one or more antagonists for the opioid releasable upon ingestion or solvent immersion, and 4) impermeable barrier means separating the opioid and the antagonist.

A transdermal dosage form comprising at least one active agent and at least one inactivating agent, wherein the inactivating agent is a cross linking agent that is released upon disruption of the dosage form is described in WO02/087482.

WO 97/04835 describes a transdermal system for administering a substance through the skin, the system including a reservoir containing the substance to be administered as an agonist as well as an associated antagonist. The system also includes at least one electrode which is polarized, when the transdermal system is used, so that only the agonist passes into the skin.

WO 03/090729 was published after the priority date of the present application. It relates to a transdermal system for administering an analgesic through the skin, the system having a reduced potential for abuse, comprising: (a) an analgesic reservoir comprising an analgesic, the analgesic being selected from the group consisting of fentanyl and analogs thereof; (b) an antagonist reservoir comprising an antagonist for the analgesic; (c) a barrier layer, the barrier layer separating the antagonist reservoir from the analgesic reservoir, the barrier layer being substantially impermeable to the analgesic and to the antagonist, wherein the system (i) substantially prevents release of the antagonist from the system upon securing the system to a human patient for a period of up to about 7 days; and (ii) provides release of the antagonist at a rate sufficient to provide an abuse limiting release rate ratio of the antagonist to the analgesic when the system is subject to abuse.

In one aspect, the present invention comprises a transdermal dosage form comprising an active agent component comprising an active agent dispersed in a polymeric material, an adverse agent component comprising an antagonist to the active agent, and a barrier. The active agent component has a proximal, skin-contacting surface, a distal surface, and at least one channel passing between the proximal and distal surfaces. The barrier is interposed between the distal surface of the active agent component and the adverse agent component. Furthermore, the barrier is permeable to a solvent selected from the group consisting of water, ethanol, ether, and mixtures thereof, and the barrier is impermeable to diffusion of active agent and adverse agent in the absence of said solvent.

In another aspect, the present invention comprises a transdermal dosage form comprising an active agent component comprising an active agent dispersed in a polymeric material, an adverse agent component comprising an antagonist to the active agent, and a discontinuous barrier. The active agent component has a proximal, skin-contacting surface, a distal surface, and at least one channel passing between the proximal and distal surfaces. The barrier is interposed between the distal surface of the active agent component and the adverse agent component. Furthermore, the barrier is impermeable to diffusion of active agent and antagonist. The dosage form comprises at least one channel passing between the skin-contacting surface and the adverse agent component.

An object of the present invention is to provide a transdermal dosage form that is resistant to abuse or misuse through extraction of active agent from the dosage form. Such extraction may be performed, for example, by complete immersion of the dosage form in a solvent, by a surface extraction of active agent from the active agent component, or by placing the dosage form in contact with a bodily fluid, such as saliva.

The above summary of the present invention is not intended to describe each disclosed embodiment or every implementation of the present invention. The Figures and the detailed description that follow more particularly exemplify illustrative embodiments.

Preferred embodiments of the invention will now be described in greater detail below with reference to the attached drawings, wherein:
FIGS. 1a, b show a schematic cross-section (1a) and a plan view (1b) of an embodiment of the present invention where the active agent component comprises strips separated by air channels.
FIGS. 2a, b show a schematic cross-section (2a) and a plan view (2b) of an embodiment of the present invention where the active agent component comprises an annular disk with a central air channel.
FIGS. 3a, b show a schematic cross-section (3a) and a plan view (3b) of an embodiment of the present invention where the active agent component comprises a disk with a plurality of cylindrical air channels.
FIGS. 4a, b show a schematic cross-section (4a) and a plan view (4b) of an embodiment of the present invention where the active agent component comprises strips separated by air channels. In this embodiment the barrier comprises strips separated by air channels and the barrier is aligned with the active agent component.
FIGS. 5a, b show a schematic cross-section (5a) and a plan view (5b) of an embodiment of the present invention where the active agent component comprises an annular disk with a central air channel. In this embodiment the barrier comprises an annular disk with a central air channel and the barrier is aligned with the active agent component.
FIGS. 6a, b show a schematic cross-section (6a) and a plan view (6b) of an embodiment of the present invention where the active agent component comprises a disk with a plurality of cylindrical air channels. In this embodiment the barrier comprises a disk with a plurality of cylindrical air channels and the barrier is aligned with the active agent component.
FIGS. 7a, b show a schematic cross-section (7a) and a plan view (7b) of an embodiment of the present invention where the active agent component comprises a disk with a plurality of cylindrical air channels. In this embodiment the barrier and adverse agent component comprise disks with a plurality of cylindrical air channels. The barrier and adverse agent component are aligned with the active agent component.
FIGS. 8a, b show a schematic cross-section (8a) and a plan view (8b) of an embodiment of the present invention where the active agent component comprises a disk with a plurality of cylindrical channels, wherein the channels comprise a dissolvable material.
FIG. 9 shows a schematic cross-section of an embodiment of the present invention where the active agent component has a structured surface facing the barrier.
FIG. 10 shows a schematic cross-section of an embodiment of the present invention where the active agent component has a structured surface facing away from the barrier.
FIG. 11 shows a schematic cross-section of an embodiment of the present invention similar as shown in FIG. 10, further comprising a structured release liner.
FIG. 12 shows a schematic cross-section of an embodiment of the present invention where the active agent component comprises dissolvable beads that provide channels between the skin-contacting surface and the barrier.
FIGS. 13a, b show a schematic cross-section (13a) and a plan view (13b) of an embodiment of the present invention where the active agent component comprises a disk with a plurality of cylindrical air channels, the barrier comprises a disk with a plurality of cylindrical air channels, and the barrier is aligned with the active agent component (as in FIG. 6a,b). In this embodiment the backing and an overlay PSA extend beyond the adverse agent component, barrier, and active agent components.
FIG. 14 shows a schematic cross-section of an embodiment of the present invention similar to that shown in FIG. 13a, except that the overlay PSA is coated uniformly across the backing, instead of being present only at the outer edges of the backing.
FIG. 15a, b show a schematic cross-section (15a) and a plan view (15b) of an embodiment of the present invention similar to that shown in FIG. 1a, b, except that a porous medium is interposed between the adverse agent component and backing layers.

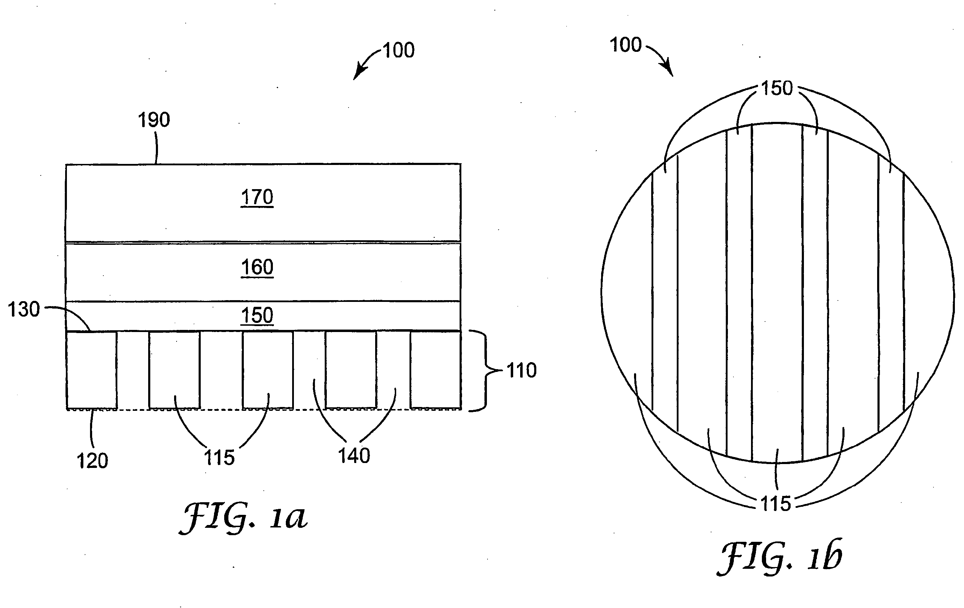

In one embodiment, shown in FIGS. 1a and 1b, the present invention comprises a transdermal dosage form 100 comprising an active agent-containing component 110 ("active agent component") comprising a skin-contacting polymeric material 115 and an active agent, an adverse agent-containing component 160 ("adverse agent component") comprising an antagonist to the active agent, and a barrier 150. The active agent component has a proximal, skin-contacting surface 120, a distal surface 130 opposed to, i.e., opposite to or in contraposition to, the proximal surface, and channels 140 passing between the proximal and distal surfaces. The barrier 150, disposed between the distal surface of the active agent component 130 and the adverse agent component 160, is present as a layer that adjoins the distal surface of the active agent component 130 and the adverse agent component 160. A backing 170 is disposed adjacent to and adjoins the adverse agent component 160 at a location which provides an outer surface 190 of the dosage form 100.

As shown in FIGS. 1a and 1b, the active agent component 110 comprises a plurality of active agent strips 115 comprising a skin-contacting polymeric material and an active agent, wherein the active agent strips are separated to define channels 140 adjacent to the strips. In this embodiment the channels 140 are filled with air. In one embodiment, the width of the active agent strips 115 is preferably more than 0.1 cm, more preferably more than 0.2 cm, and most preferably more than 0.4 cm. In another embodiment, the width of the active agent strips is preferably less than 2.0 cm, more preferably less than 1.0 cm, and most preferably less than 0.6 cm.

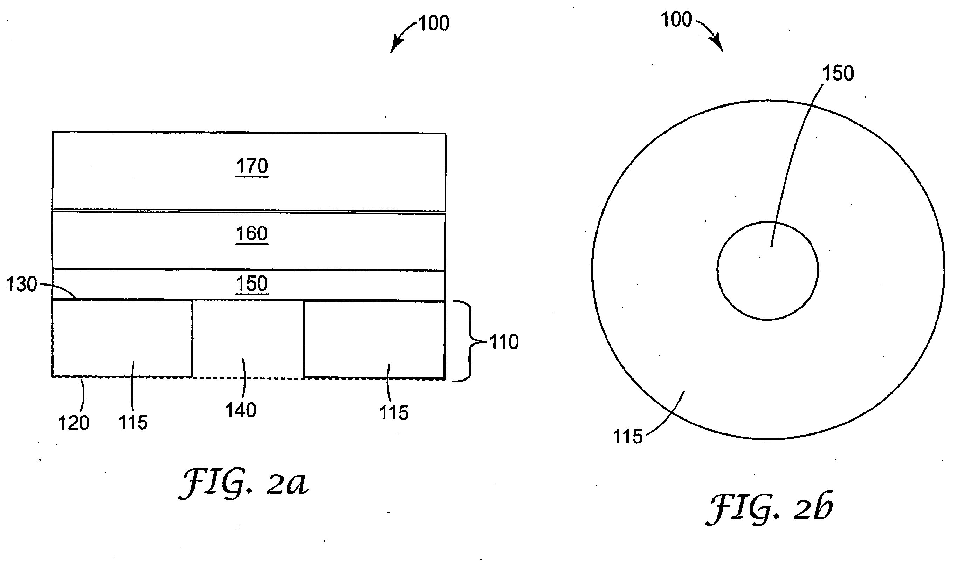

In another aspect, as shown in FIGS. 2a and 2b, the active agent component 110 consists of an annular disk comprising a skin-contacting polymeric material 115 and an active agent which is configured to define a central channel 140 filled with air.

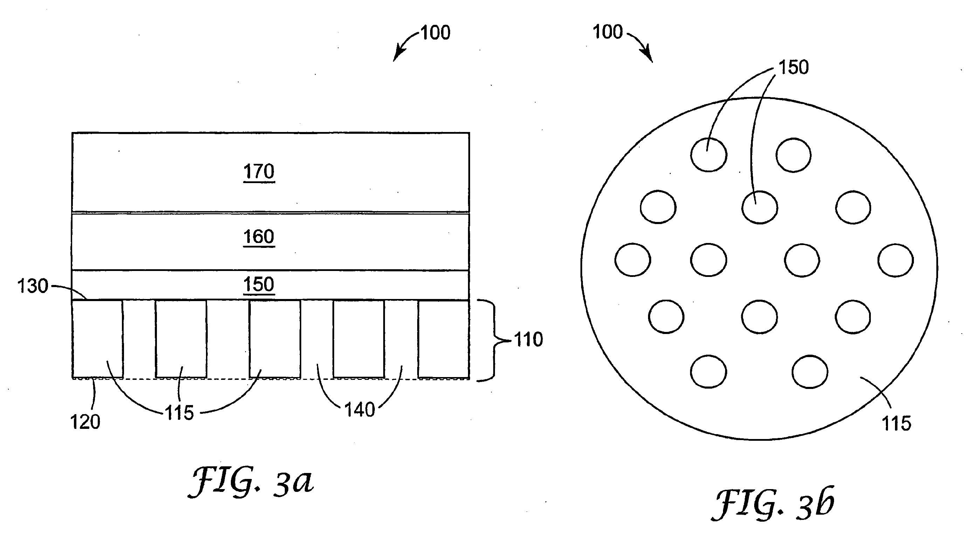

In still another aspect, as shown in FIGS. 3a and 3b, the active agent component 110 comprises a polymeric disk configured to define a plurality of cylindrical air channels 140. In one embodiment, the diameter of the cylindrical air channels 140 is preferably more than 0.015 cm, more preferably more than 0.05 cm, and most preferably more than 0.1 cm. In another embodiment, the diameter of the cylindrical air channels 140 is preferably less than 1.0 cm, more preferably less than 0.5 cm, and most preferably less than 0.2 cm.

In each of these aspects, the total surface area of the channels 140 is preferably more than 0.5 %, more preferably more than 1 %, and most preferably more than 2 % of the total surface area of the skin-contacting surface 120. The total surface area of the channels 140 is preferably less than 40%, more preferably less than 20%, and most preferably less than 10 % of the total surface area of the skin-contacting surface 120.

Although several specific configurations are described above, it should be understood that the channels may be of any shape, for example, squares, diamonds, or ovals. The channels may also be interconnected leaving islands or peninsulas of skin-contacting polymeric material within the active agent component.

The active agent component comprises a skin-contacting polymeric material and an active agent. The active agent is preferably dispersed homogeneously throughout the skin-contacting polymeric material, and more preferably dissolved within the skin-contacting polymeric material. The proximal, skin-contacting surface should be sufficiently conformable when placed on a skin surface so as to make intimate contact with at least a portion of the skin surface. In one aspect, substantially all of the skin-contacting polymeric material at the skin-contacting surface will make intimate contact with the skin surface. In one embodiment, the active agent component will preferably have a thickness of no less than 10 µm, more preferably no less than 20 µm, and most preferably no less than 50 µm. In another embodiment, the skin contact layer will preferably have a thickness of no more than 250 µm, more preferably no more than 200 µm, and most preferably no more than 150 µm.

The skin-contacting polymeric material comprises a polymer, preferably a polymer selected from the group consisting of acrylates, natural rubbers, synthetic rubbers, such as polyisobutylenes, polyisoprenes, styrenic block copolymers, polyvinylethers, silicone polymers, polyurethanes, and polyurethane-ureas. The polymers can be present alone or in combination. The skin-contacting polymeric material may optionally contain other additives, for example, penetration enhancers, tackifiers, plasticizers, anti-oxidants, colorants, crystallization inhibitors, and the like.

In one aspect, the skin-contacting polymeric material may comprise a pressure-sensitive adhesive. Preferred pressure-sensitive adhesives for use in dosage forms of the invention include acrylates, polyisobutylenes, silicone polymers, and mixtures thereof. Examples of useful polyisobutylene pressure-sensitive adhesives are described in U.S. Patent No. 5,985,317 (Venkateshwaran et al.). Examples of useful acrylate and silicone polymer pressure-sensitive adhesives, and mixtures thereof, are described in U.S. Patent No. 5,474,783 (Miranda).

Acrylate polymers and copolymers are particularly preferred pressure-sensitive adhesives. Examples of suitable monomers for use in acrylate copolymers include alkyl acrylates, such as isooctyl, 2-ethylhexyl, n-butyl, ethyl, methyl, and dimethylhexyl, and alkyl methacrylates, such as lauryl, isodecyl, and tridecyl. Monomers containing functional groups, such as carboxylic acid, hydroxy, amide, and amino may also be incorporated into an acrylate copolymer. Examples of suitable monomers containing functional groups include acrylic acid, hydroxyalkyl acrylates containing 2 to 4 carbon atoms in the hydroxyalkyl group, acrylamide, N-vinyl-2-pyrrolidone, vinyl acetate, and alkoxyethyl acrylates.

Acrylate copolymers may optionally further comprise a substantially linear macromonomer copolymerizable with the other monomers. Suitable macromonomers include polymethylmethacrylate, styrene/acrylonitrile copolymer, polyether, and polystyrene macromonomers. Examples of useful macromonomers and their preparation are described in U.S. Patent No. 4,693,776 (Krampe et al.).

The active agent of the present invention may be any drug substance that is capable of being abused. Many drugs have a potential for abuse, and include but are not limited to, for example, narcotics, such as morphine, fentanyl, meperidine, codeine, sufentanil, and oxycodone; psychostimulants, such as amphetamine, methamphetamine, and methylphenidate; methoxy substituted amphetamines, such as 3,4-methylenedioxymethamphetamine (MDMA); and benzodiazepines, such as diazepam, oxazepam, and lorazepam.

The active agent or drug will be present in an amount such that the composition delivers a therapeutically effective amount for the condition being treated. This amount will vary according to the type of drug used, the condition to be treated, the amount of time the composition is allowed to remain in contact with the skin of the subject, and other factors known to those of skill in the art. For example, information on dosing and the amount of opioid agonist active agent present in a transdermal dosage form is set forth in U.S. Published Patent Application No. 2002/0119187 A1, filed September 26, 2001, entitled "Composition for the Transdermal Delivery of Fentanyl" by Cantor et al. and U.S. Published Patent Application No. 2003/0026829 A1, filed March 15, 2002, entitled "Transdermal Administration of Fentanyl and Analogs Thereof" by Venkatraman et al. In one embodiment, the amount of active agent present in the transdermal drug delivery composition of the invention is greater than about 0.01 wt-% and preferably greater than about 1.0 wt-%, based on the total weight of the composition. In another embodiment, the amount of active agent present in the transdermal drug delivery composition of the invention is less than about 40 wt-% and preferably less than about 20.0 wt-%, based on the total weight of the composition.

The analgesically effective amount of an opioid present in the transdermal dosage form, however, typically ranges from about 0.01 to about 50 mg/cm² in one embodiment, from about 0.05 to about 15 mg/cm² in another embodiment, and from about 0.05 to about 5.0 mg/cm² in another embodiment. It is well within the purview of one skilled in the art to readily determine the analgesically effective amount of an opioid needed for a particular indication.

In FIGS. 1, 2, and 3 the adverse agent component 160 is connected on one side to a barrier 150 component and on the other side to a backing 170. The adverse agent component 160 can be a polymeric material, porous film, or other material suitable for containing an antagonist to the active agent. Preferably, the adverse agent component 160 is be capable of containing sufficient antagonist to blunt or block at least one biological effect of the active agent or to cause at least one unpleasant side effect in a patient or animal which has absorbed the total amount of active agent in the dosage form 100. This amount can vary according to the type of antagonist used, the amount and type of active agent used, and the mode of abuse.

Furthermore, the adverse agent component 160 should be capable of releasing antagonist when it comes into contact with extraction solvents, such as water, ethanol, ether, or mixtures thereof.

Suitable polymeric materials for use in the adverse agent component include acrylates, natural rubbers, synthetic rubbers such as polyisobutylenes, polyisoprenes, styrenic block copolymers, polyvinylethers, silicone polymers, polyurethanes, and polyurethane-ureas. The antagonist is preferably dispersed homogeneously throughout the polymeric material. In one aspect, the antagonist is dissolved within the polymeric material. In another aspect, solid crystals of antagonist are dispersed throughout the polymeric material. Preferably, the polymeric material is a pressure sensitive adhesive. Suitable pressure-sensitive adhesives include those suitable for use as a skin-contacting polymeric material. Additionally, pressure-sensitive adhesives that are not suitable for direct skin contact can be suitable for use as the polymeric material of the adverse agent component.

The adverse agent component can also be a porous medium, such as a woven fabric, porous or microporous film, or other open, mesh-like material, wherein the pores are at least partially filled with antagonist. The antagonist can be present within the pores as a solid crystalline or powdered material. Alternatively, the antagonist may be mixed with a carrier, such as a viscous liquid or semi-solid material. Examples of suitable films include, for example, microporous films formed by extruding polyethylene or polypropylene with mineral oil as described in U.S. Patent No. 4,539,256 (Shipman).

The antagonist to the active agent is a compound or composition that acts to prevent, diminish, or delay the pharmacological effects of the active agent, or otherwise acts to deter potential abuse. Antagonists may include, for example, narcotic antagonists, such as naltrexone, naloxone, and nalbuphine; bitter tasting substances; emetics, or nauseants. Narcotic antagonists, most preferably naltrexone, are preferably used in conjunction with abusable narcotics. The antagonist will preferably act to blunt or block at least one biological effect of the active agent or to cause at least one unpleasant side effect in a patient or animal which has absorbed the active agent.

The barrier 150, as shown in FIGS. 1, 2, and 3, is a substantially continuous component adjacent to and adjoining the distal surface of the active agent component 130 on one side and the adverse agent component 160 on the other side. The barrier is permeable to a solvent selected from the group consisting of water, ethanol, ether, and mixtures thereof, and the barrier is impermeable to diffusion of active agent and antagonist in the absence of said solvent.

In relation to the present invention, permeability of the barrier to a solvent is defined such that solvent may pass through the barrier or that the solvent may dissolve or erode the barrier, such that drug and/or antagonist can pass through the barrier in the presence of solvent. Movement of drug and/or antagonist across the barrier will occur in amounts and over a time period that will vary depending on the particular application for the dosage form, as well as the relative amounts and types of drug and antagonist, but it will be understood that sufficient antagonist will pass through the barrier in the presence of solvent to have an attenuating effect on the abuse potential of an amount of active agent that may be co-extracted by the solvent. It is preferred that antagonist will pass through the barrier after the barrier has been exposed to solvent for a time period of less than 30 minutes, more preferably less than 15 minutes, and most preferably less than 5 minutes. The amount of antagonist that will pass through the barrier is preferably more than 10 µg. more preferably more than 50 µg, and most preferably more than 200 µg.

Although extraction may be performed in a laboratory type setting (e.g., by immersion of a dosage form in a beaker of solvent), it should also be understood that extraction of antagonist and active agent may also take place in vivo, i.e., in a physiological setting, such as in the saliva present in the oral cavity or the gastric fluid present in the stomach.

The impermeability of the barrier to diffusion of active agent and antagonist in the absence of a solvent is such that less than significant amounts, and preferably none, of active agent or antagonist diffuse across the barrier during normal storage or use of the dosage form. The precise amounts which are less than significant will vary depending on the particular composition and intended therapeutic application of the dosage form, but it will be understood to include any amounts of active agent or antagonist that do not significantly alter the therapeutic effect of the dosage form (e.g., the active agent concentration in the active agent component does not change significantly due to diffusion of active agent across the barrier and a pharmacologically effective amount of antagonist does not diffuse across the barrier and into the active agent component). Any insignificant amounts of active agent that may diffuse across the barrier are preferably less than 5%, more preferably less than 1%, and most preferably less than 0.1 % by weight of the total drug in the dosage form. Any insignificant amounts of drug that may diffuse across the barrier will preferably do so over a time period greater than 1 month, more preferably greater than 6 months, and most preferably greater than 2 years.

In a preferred embodiment, the active agent is not in diffusional communication with the adverse agent component. Diffusional communication is understood to mean that a substance, such as a drug, is able to diffuse from one area to another by passing through or across one or more solid or liquid media.

Suitable barriers may include, for example, dissolvable films, such as polyvinyl alcohol or modified polyvinyl alcohols, for example, polyvinyl alcohol with additional material blended in. Suitable barriers may also include porous or microporous films.

The barrier thickness is preferably more than 1 µm, more preferably more than 10 µm, and most preferably more than 20 µm. The barrier thickness is preferably less than 100 µm, more preferably less than 75 µm, and most preferably less than 50 µm.

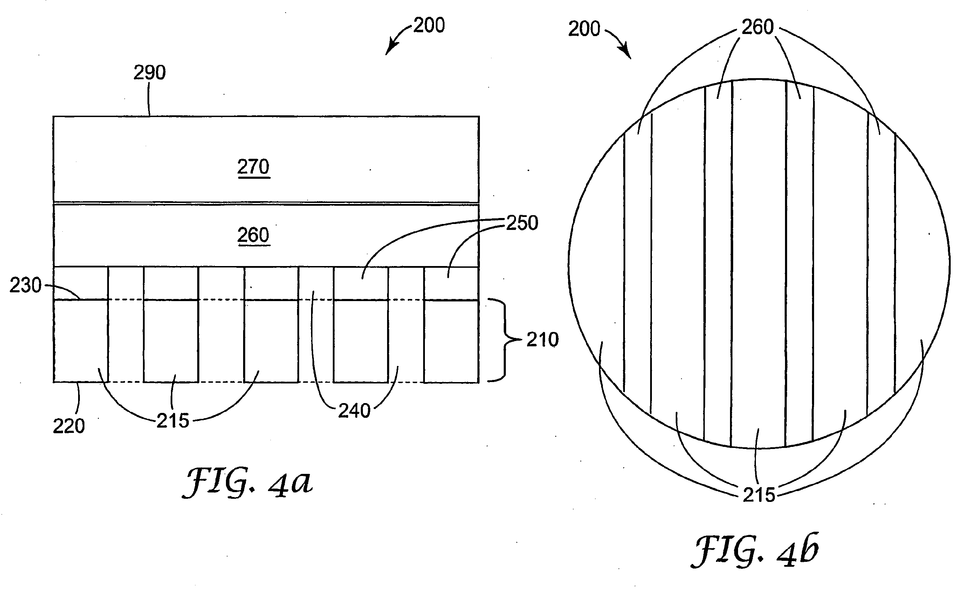

In another embodiment, shown in FIGS. 4a and 4b, the present invention comprises a transdermal dosage form 200 comprising an active agent component which is a skin-contacting component 210 comprising a skin-contacting polymeric matrix 215 and an active agent, a reservoir or adverse agent component 260 comprising an antagonist to the active agent, and a barrier 250. The skin-contacting component has a proximal, skin-contacting surface 220, a distal surface 230 opposed to the skin-contacting surface, and channels 240 passing between the proximal and distal surfaces. The barrier 250 is present as a discontinuous component that is adjacent to and adjoins the distal surface of the skin-contacting component 230 and the reservoir component 260. A backing 270 is adjacent to and adjoins the surface of the reservoir 260 and provides an outer surface 290 of the dosage form 200.

As shown in FIGS. 4a and 4b, the skin-contacting component 210 comprises a plurality of strips comprising a skin-contacting polymeric matrix 215 and an active agent, wherein the strips are separated by channels 240. In this embodiment the channels 240 are filled with air. The discontinuous barrier is aligned with the skin-contacting polymeric material 215 so that at least one continuous air channel 240 passes from a plane defined by the adjacent proximal, skin-contacting surface 220 to the reservoir component 260. In one embodiment, the width of the strips comprising the skin-contacting polymeric material 215 and the active agent is greater than 0.1 cm, preferably greater than 0.2 cm, and more preferably greater than 0.4 cm. In another embodiment, the width of the strips comprising the skin-contacting polymeric matrix 215 and the active agent is less than 2.0 cm, preferably less than 1.0 cm, and more preferably less than 0.6 cm.

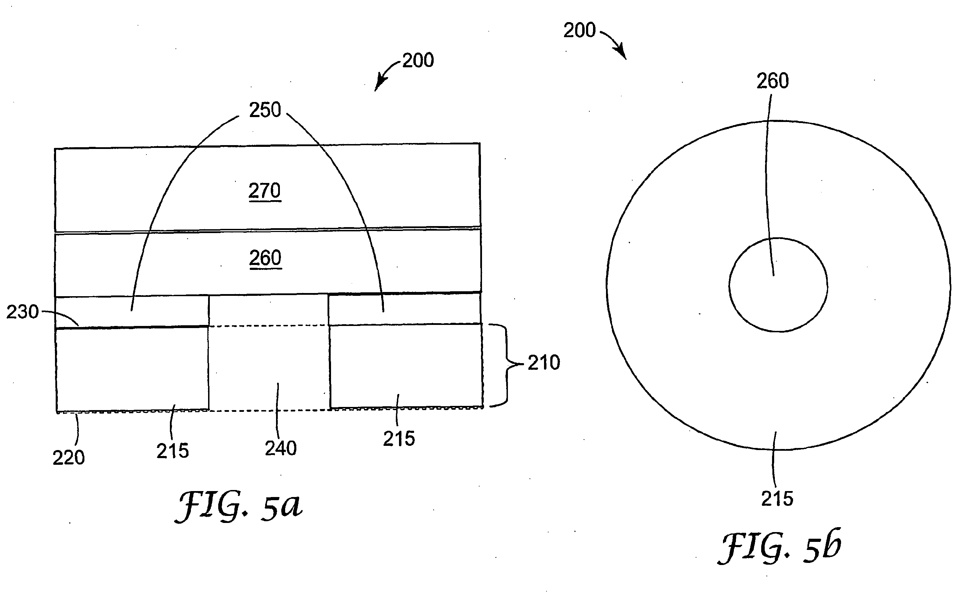

In another aspect, as shown in FIGS. 5a and 5b, the active agent or skin-contacting component 210 consists of an annular disk comprising a skin-contacting polymeric matrix 215 and an active agent with a central channel 240 filled with air.

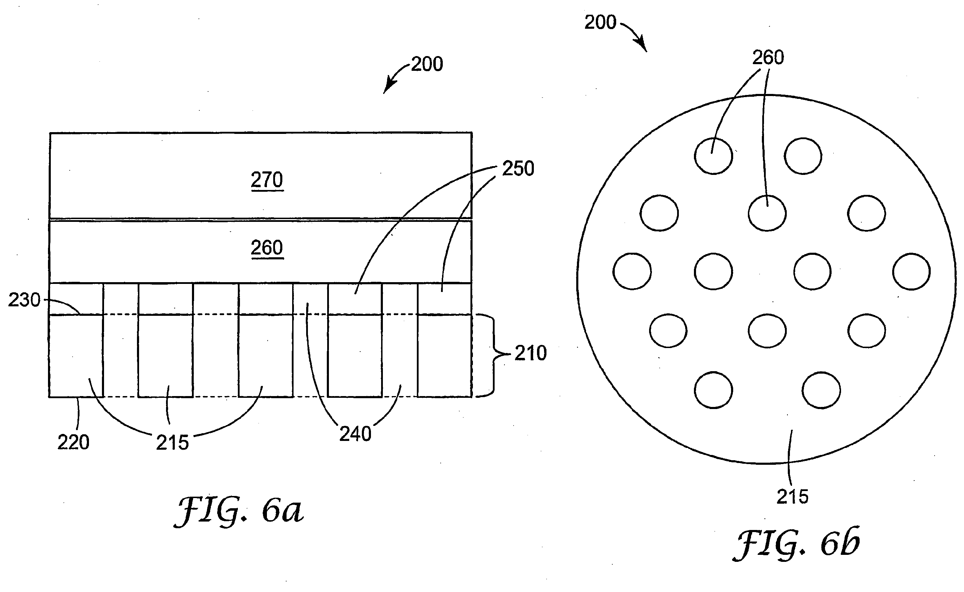

In still another aspect, as shown in FIGS. 6a and 6b, the active agent or skin-contacting component 210 consists of a disk with a plurality of cylindrical air channels 240. In one embodiment, the diameter of the cylindrical air channels 240 is greater than 0.015 cm, preferably greater than 0.05 cm, and more preferably greater than 0.1 cm. In another embodiment, the diameter of the cylindrical air channels 240 is less than 1.0 cm, preferably less than 0.5 cm, and more preferably less than 0.2 cm.

In each of these aspects, the total surface area of the channels 240 is preferably more than 0.5 %, more preferably more than 1%, and most preferably more than 2 % of the total surface area of the skin-contacting surface 220. The total surface area of the channels 240 is preferably less than 40%, more preferably less than 20%, and most preferably less than 10 % of the total surface area of the skin-contacting surface 220.

The barrier 250, as shown in FIGS. 4, 5, and 6, is a discontinuous component adjacent to and adjoining the distal surface of the active agent or skin-contacting component 230 on one side and the adverse agent or reservoir component 260 on the other side. The barrier is impermeable to diffusion of active agent and antagonist.

The impermeability of the barrier to diffusion of active agent and antagonist is such that less than significant amounts, and preferably none, of active agent or antagonist diffuse across the barrier during normal storage or use of the dosage form. The precise amounts which are less than significant will vary depending on the particular composition and intended therapeutic application of the dosage form, but it will be understood to include any amounts of active agent or antagonist that do not significantly alter the therapeutic effect of the dosage form (e.g., the active agent concentration in the active agent component does not change significantly due to diffusion of active agent across the barrier and a pharmacologically effective amount of antagonist does not diffuse across the barrier and into the active agent component). Any insignificant amounts of active agent that may diffuse across the barrier are preferably less than 5%, more preferably less than 1%, and most preferably less than 0.1% by weight of the total drug in the dosage form. Any insignificant amounts of drug that may diffuse across the barrier will preferably do so over a time period greater than 1 month, more preferably greater than 6 months, and most preferably greater than 2 years.

Suitable barriers for use as a discontinuous component can be prepared from films comprised of, for example, polyesters, such as polyethylene terephthalate; polypropylenes; and polyethylenes, such as high density polyethylene. Multi-layered films, such as polyethylene terephthalate-aluminum-polyethylene composites or polyethylene terephthalate-ethylene vinyl acetate composites are also suitable.

The barrier thickness is preferably more than 1 µm, more preferably more than 10 µm, and most preferably more than 20 µm. The barrier thickness is preferably less than 100 µm, more preferably less than 80 µm, and most preferably less than 60 µm.

Dissolvable films, such as the films described in the embodiments shown in FIGS. 1, 2, and 3 may be optionally used.

Barriers of the present invention may be formed having a discontinuous structure that is subsequently laminated or otherwise attached to the discontinuous structure of the active agent component. It is preferred that the barrier and the skin-contacting polymeric material or matrix are fully aligned. It is not necessary for the two layers to be completely registered, however, as long as the barrier serves to deter diffusion of the active agent and antagonist through the barrier layer. Discontinuous barriers of the present invention may also be formed at the same time that the discontinuities in the skin-contacting polymeric material are formed. For example, a continuous barrier film may be coated with a continuous skin-contacting polymeric matrix or laminated to a continuous skin-contacting polymeric matrix. Apertures or holes may be created in the laminate using any suitable hole-forming process, such as punching, so that aligned channels are simultaneously created in both the barrier and the skin-contacting polymeric material.

Barriers of the present invention can also comprise an impermeable surface coating applied to one of the other surfaces present in the dosage form, such as the distal surface of the active agent component opposed to the skin-contacting surface or the surface of the adverse agent component facing the active agent component. Examples of suitable coatings include fluoropolymers, such as polymers or copolymers of tetrafluoroethylene, hexafluoropropylene, and/or vinylidene fluoride. Terpolymers of tetrafluoroethylene, hexafluoropropylene, and vinylidene fluoride, such as Dyneon^{™} fluorothermoplastic THV are preferred coatings. The thickness of impermeable surface coatings is preferably between 0.5 and 10 µm thick, more preferably between 1 and 5 µm thick, and most preferably between 2 and 4 µm thick. In one aspect, the barrier is a thin coating on the surface of an adverse agent component comprising a microporous film.

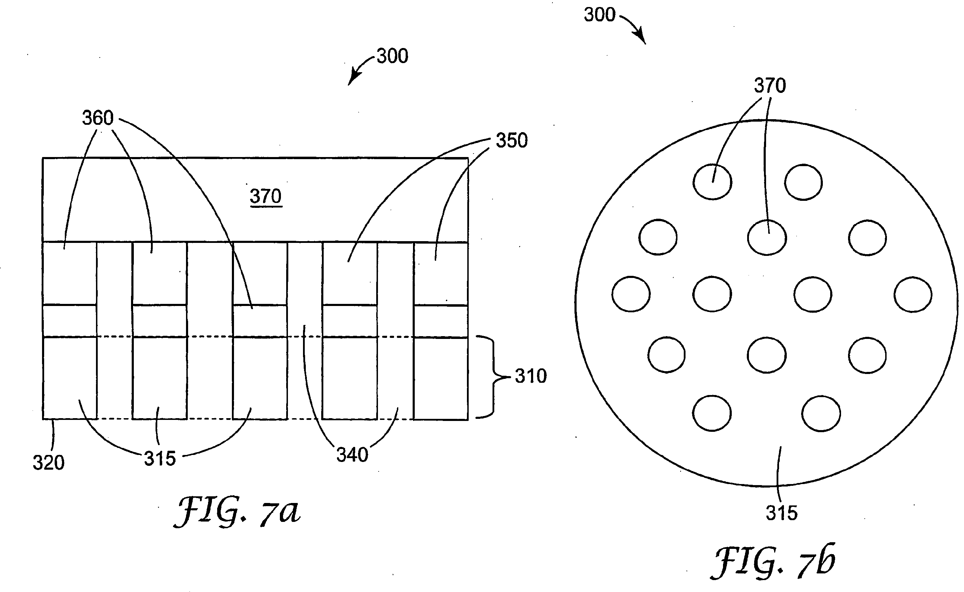

Channels 340 may also be present in the adverse agent or reservoir component 360. As shown in FIGS. 7a, and 7b the channels 340 are a plurality of apertures that are substantially aligned with the channels in the barrier 350 and skin-contacting polymeric matrix 315. Channels in the reservoir component 360 may be formed in the same manner as channels in the other components, that is, independently with a subsequent lamination/alignment step or simultaneously following lamination of continuous components.

In the embodiments shown in FIGS. 4, 5, 6, and 7 suitable skin-contacting components, skin-contacting polymeric matrices, active agents, reservoir components, and antagonists are as described in the embodiments shown in FIGS. 1,2, and 3.

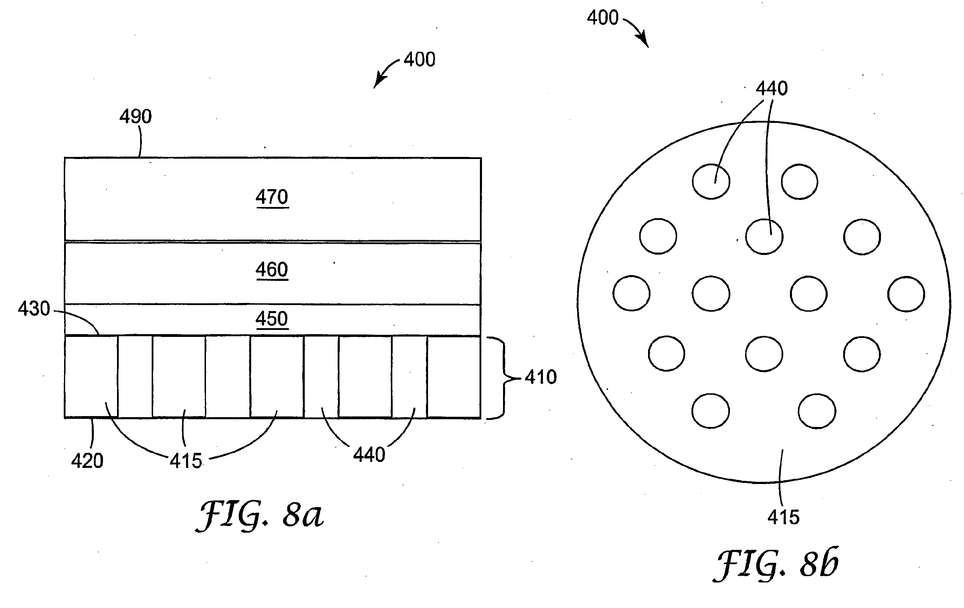

In another embodiment, shown in FIGS. 8a and 8b, the present invention comprises a transdermal dosage form 400 comprising an active agent component which is a skin-contacting component 410 comprising a skin-contacting polymeric matrix 415 and an active agent, an adverse agent or reservoir component 460 comprising an antagonist to the active agent, and a barrier 450. The skin-contacting component has a proximal, skin-contacting surface 420, a distal surface 430 opposed to the skin-contacting surface, and channels 440 passing between the proximal and distal surfaces. The barrier 450 is present as a component that is adjacent to and adjoins the distal surface of the skin-contacting component 430 and the reservoir component 460. A backing 470 is adjacent to and adjoins the adverse agent component 460 and provides an outer surface 490 of the dosage form 400.

As shown in FIGS. 8a and 8b, the skin-contacting component 410 consists of a disk with a plurality of cylindrical channels 440. The channels 440 are filled with a dissolvable, erodible, or porous material, such that the channels 440 are permeable to a solvent selected from the group consisting of water, ethanol, ether, and mixtures thereof, and the channels 440 are impermeable to diffusion of active agent and antagonist in the absence of said solvent. Suitable materials for use as channels 440 may include, for example, dissolvable films, such as polyvinyl alcohol or modified polyvinyl alcohols. Suitable materials may, also include porous or microporous films.

In one embodiment, the diameter of the cylindrical channels 440 is greater than 0.015 cm, preferably greater than 0.05 cm, and more preferably greater than 0.1 cm. In another embodiment, the diameter of the cylindrical air channels 440 is less than 1.0 cm, preferably less than 0.5 cm, and more preferably less than 0.2 cm.

In each of these aspects, the total surface area of the channels 440 is preferably more than 0.5 %, more preferably more than 1%, and most preferably more than 2 % of the total surface area of the skin-contacting surface 420. The total surface area of the channels 440 is preferably less than 40%, more preferably less than 20%, and most preferably less than 10 % of the total surface area of the skin-contacting surface 420.

In the embodiment shown in FIG. 8, suitable skin-contacting components, skin-contacting polymeric matrices, active agents, adverse agent components, barriers, and antagonists are as described in the embodiments shown in FIGS. 1, 2, and 3.

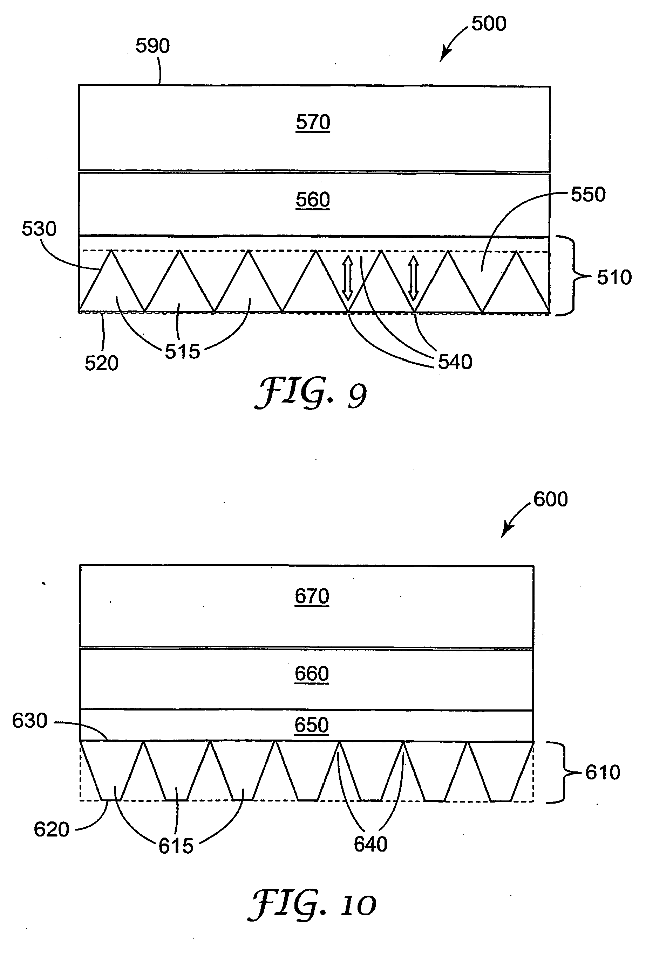

In another embodiment, shown in FIG. 9, the present invention comprises a transdermal dosage form 500 comprising an active agent component which is a skin-contacting component 510 comprising a skin-contacting polymeric matrix 515 and an active agent, an adverse agent or reservoir component 560 comprising an antagonist to the active agent, and a barrier 550. The skin-contacting component has a proximal, skin-contacting surface 520 and a distal surface 530 opposed to the skin-contacting surface. As shown in FIG. 9, the distal surface 530 opposed to the skin-contacting surface is a structured surface composed of a series of ridges or alternatively pyramids. The barrier 550 is present as a component that is adjacent to and adjoins the distal surface of the skin-contacting component 530, and as such has an interlocking pattern of ridges or pyramids. The non-patterned side of the barrier adjoins the reservoir component 560. A backing 570 adjoins the reservoir component 560 and provides an outer surface 590 of the dosage form 500.

The ridges or pyramids may be formed by any well known techniques for preparing embossed or microreplicated polymeric layers, such as those in U.S. Patent No. 6,123,890 (Mazurek et al.).

Suitable barriers include barrier materials of the embodiments described in FIGS. 1, 2, and 3.

The channels 540 are formed connecting the skin-contacting surface 520 to the reservoir component 560. As shown in FIG. 9, the channels 540 are filled with the barrier 550. The barrier is permeable to and/or at least partially soluble in a solvent selected from the group consisting of water, ethanol, ether, and mixtures thereof, and the barrier is substantially impermeable to diffusion of active agent and adverse agent in the absence of said solvent. The ridges or pyramids of the skin-contacting polymeric matrix 515 are preferably formed such that a small opening exists between neighboring ridges or pyramids. As shown in FIG. 9, the ridges or pyramids abut each other at a point contact. It should be understood that a point contact between neighboring ridges can effectively allow for fluid communication of solvent between the skin-contacting surface 520 and the reservoir component 560. It should be further understood that the channel may contain an insubstantial amount of the skin-contacting polymeric material, but can still substantially provide an open passage to solvent. The connection between neighboring ridges or pyramids is of an insubstantial amount, preferably with a thickness of less than 5 µm, and more preferably less than 1 µm.

In this embodiment, suitable skin-contacting components, skin-contacting polymeric matrices, active agents, adverse agent components, barriers, and antagonists are as described in the embodiments shown in FIGS. 1, 2, and 3.

In another embodiment, shown in FIG. 10, the present invention comprises a transdermal dosage form 600 comprising an active agent component which is a skin-contacting component 610 comprising a skin-contacting polymeric matrix 615 and an active agent, an adverse agent or reservoir component 660 comprising an antagonist to the active agent, and a barrier 650. The skin-contacting component has a proximal, skin-contacting surface 620, a distal surface 630 opposed to the skin-contacting surface, and channels 640 passing between the proximal and distal surfaces. As shown in FIG. 10, the skin-contacting polymeric matrix 615 is a structured component comprising ridges or truncated pyramids.

The channels 640 provide open fluid communication between the skin-contacting surface 620 to the barrier 650. The ridges or pyramids of the skin-contacting component 610 are preferably formed such that a small opening exists between neighboring ridges or pyramids. As shown in FIG. 10, the ridges or pyramids abut each other at a point contact. It should be understood that a point contact between neighboring ridges can effectively allow for fluid communication of solvent between the skin-contacting surface 620 and the barrier 650. It should be further understood that the channel may contain an insubstantial amount of the skin-contacting polymeric matrix, but can still substantially provide an open passage to solvent. The connection between neighboring ridges or pyramids is of an insubstantial amount, preferably with a thickness of less than 5 µm, and more preferably less than 1 µm.

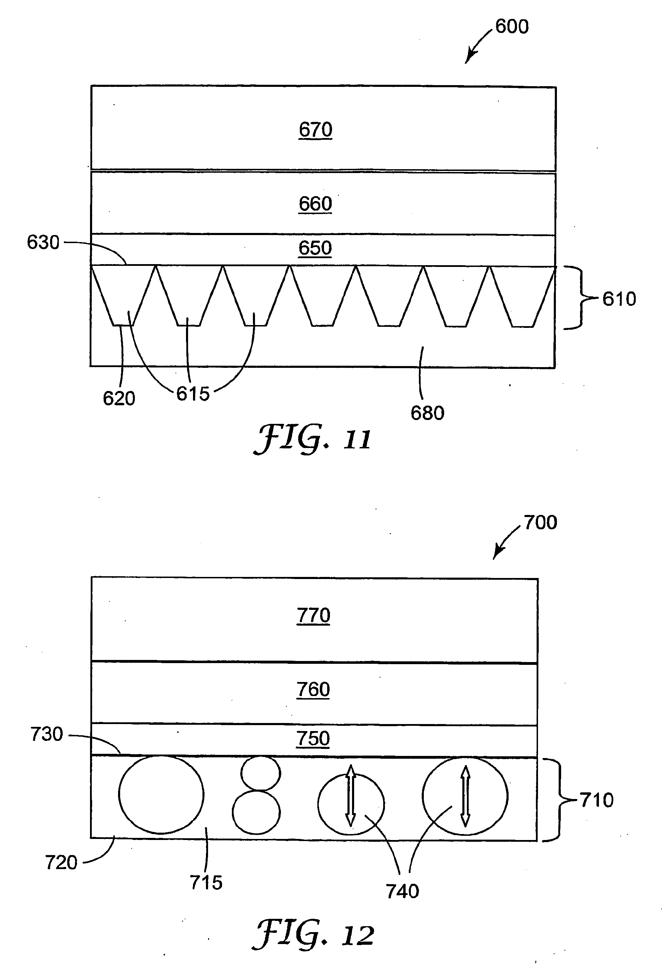

As shown in FIG. 11, the dosage form of FIG. 10 may further comprise a structured release liner 680 which serves to protect the structured surface of the skin-contacting polymeric material 615 prior to use of the dosage form.

In this embodiment, suitable skin-contacting components, skin-contacting polymeric matrices, active agents, reservoir components, barriers, and antagonists are as described in the embodiments shown in FIGS. 1, 2, and 3.

In another embodiment, shown in FIG. 12, the present invention comprises a transdermal dosage form 700 comprising an active agent component which is a skin-contacting component 710 comprising a skin-contacting polymeric matrix 715 and an active agent, an adverse agent or reservoir component 760 comprising an antagonist to the active agent, and a barrier 750. The skin-contacting component has a proximal, skin-contacting surface 720, a distal surface 730 opposed to the skin-contacting surface, and channels 740 passing substantially entirely between the proximal and distal surfaces. The channels 740 connecting the skin-contacting surface 720 to the reservoir component 760 are formed from one or more dissolvable, erodible, or porous beads imbedded within the skin-contacting polymeric material 715. One or more beads may extend completely through the skin-contacting component 710. Alternatively, one or more beads may be adjacent to each other so as to effectively provide a channel through the active agent component 710 in the presence of solvent. The beads preferably extend from the proximal, skin-contacting surface 720 to the distal surface opposed to the skin-contacting surface 730, but it should be understood that insubstantial amounts of the skin-contacting polymeric material may be present on one or both sides of the bead(s). Thus, the channel may contain a small amount of the skin-contacting polymeric matrix 715, but will still provide substantially open passage to solvent. The amount of skin-contacting polymeric material 715 within the channel 740 is of an insubstantial amount, preferably with a thickness of less than 5 µm, and more preferably less than 1 µm.

A number of optional features may be included with any one of the previously described embodiments.

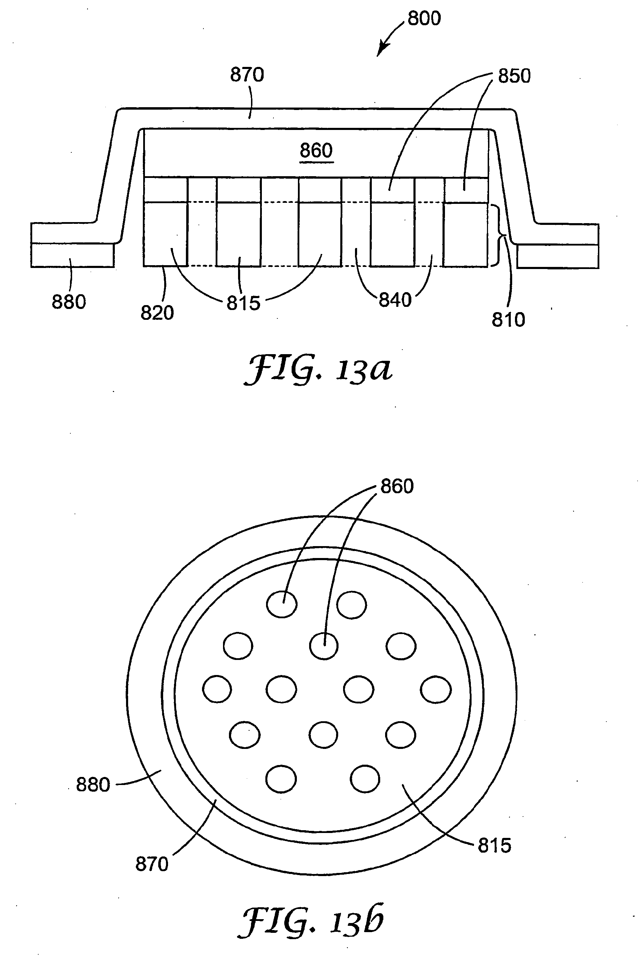

As shown in FIGS. 13a and 13b, an overlay backing 870 extends beyond the area of the adverse agent or reservoir component 860, barrier 850, and active agent or skin-contacting component 810 to a sufficient extent to allow the peripheral edge of overlay backing 870 to contact the skin surface of a patient.

The edges of the overlay backing 870 are coated with an overlay pressure sensitive adhesive (PSA) 880 that is used to secure the edges of the overlay backing 870 to a skin surface. Any pressure sensitive adhesive suitable for use in skin-contacting applications, as previously described, can be used as the overlay PSA 880. Typical examples of flexible backing materials employed as conventional tape backings which may be useful for the present invention include those made polymer films such as polypropylene; polyethylene, particularly low density polyethylene, linear low density polyethylene, metallocene polyethylenes, and high density polyethylene; polyvinyl chloride; polyester (e.g., polyethylene terephthalate); ethylene-vinyl acetate copolymer; polyurethane; cellulose acetate; and ethyl cellulose. Backings that are layered, such as polyethylene terephthalate-aluminum-polyethylene composites, are also suitable. Fabrics and non-wovens are also suitable. In a preferred embodiment, the overlay backing is a continuous polymeric film that prevents ingress of external moisture into the adverse agent component from activities such as showering and bathing. Examples of such continuous films include polyurethane, polyethylene, and polyester.

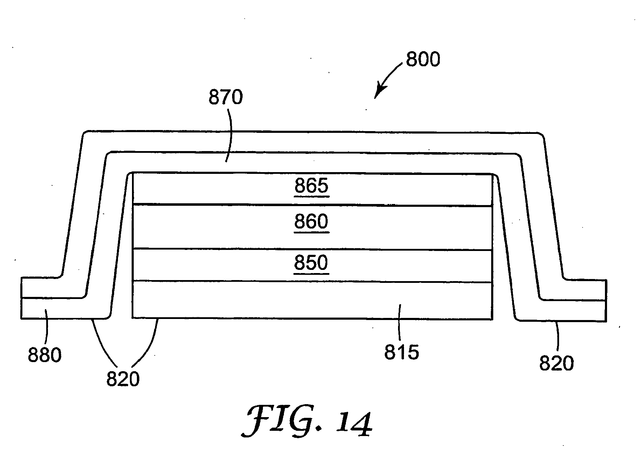

As shown in FIG. 14, the overlay backing 870 is continuously coated with an overlay pressure sensitive adhesive (PSA) 880 that is used to secure the edges of the overlay backing 870 to a skin surface. An additional, optional feature is a porous medium 865 included between the reservoir 860 and the overlay PSA 880. In this embodiment, the overlay PSA 880 serves a dual purpose. The area of the overlay PSA 880 extending beyond the area of the reservoir component 860, barrier 850, and active agent or skin-contacting component 810 serves to secure the dosage form to a skin surface and defines air channel 890. The area of the overlay PSA 880 that does not extend beyond the reservoir component 860 provides secure lamination of the overlay backing 870 to the porous medium 865 (or alternatively to the reservoir component 860 in dosage forms that do not have a porous layer).

The porous medium 865 can be any porous medium, such as a woven fabric, microporous film, or other open, mesh-like material. If the dosage form 800 is immersed in a solvent bath, then the porous medium 865 allows for fluid communication of the solvent with the top surface of the adverse agent component 860. Examples of dosage forms incorporating a porous medium between the adverse agent component and the overlay are described in U.S. Patent Application Serial No. 10/744,996, filed December 23, 2003, entitled "Abuse-Resistant Transdermal Dosage Form" by Hart et al.

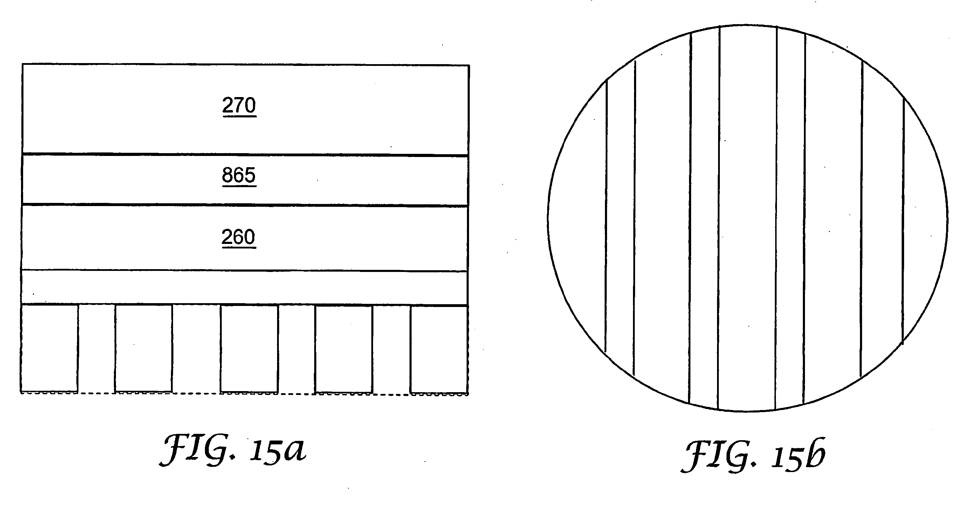

The porous medium 865 can be used in conjunction with an overlay backing 870, but it is not necessary to combine these optional features. For example, the porous medium 865 may also be present in a dosage form as shown in FIGS. 15a and 15b, where all of the features are the same as shown in FIGS. 1a and 1b, except that a porous medium 865 is inserted between the backing 170 and adverse agent component 160.

The active agent component, which may be a skin-contacting component, may comprise a number of additional components in addition to a polymeric material or matrix and an active agent. Additional components of the active agent component can include skin penetration enhancers, drug solubilizers, plasticizers, anti-oxidants, colorants, and the like.

Examples of excipients useful as skin penetration enhancers or solubilizers in transdermal drug delivery systems include C₈-C₂₄ fatty acids such as isostearic acid, octanoic acid, and oleic acid; C₈-C₂₄ fatty alcohols such as oleyl alcohol and lauryl alcohol; lower alkyl esters of C₈-C₂₄ fatty acids such as ethyl oleate, isopropyl myristate, butyl stearate, and methyl laurate; monoglycerides of C₈-C₂₄ fatty acids such as glyceryl monolaurate; tetraglycol (tetrahydrofurfuryl alcohol polyethylene glycol ether); tetraethylene glycol (ethanol,2,2'-(oxybis(ethylenoxy))diglycol); polyethylene glycol; propylene glycol; N,N-dimethyldodecylamine-N-oxide; terpenes, such as d-limonene, menthol, and terpineol.

In compositions of the active agent component of the present invention the skin penetration enhancers, drug solubilizers, plasticizers, and other additives are dispersed, preferably substantially uniformly, and more preferably dissolved in the composition. Where the additive is a penetration enhancer, it is present in an amount that enhances drug permeation through the skin compared to a like composition not containing the penetration enhancer(s) when this phenomenon is measured using a standard skin penetration model, such as in U.S. Patent No. 5,585,111 (Peterson). The total amount of penetration enhancer and solubilizer will generally be less than 40% by weight, preferably less than 30% based on the total weight of the composition.

Active agent or skin-contacting component compositions of the invention can be prepared by combining the polymer matrix, active agent, and optional additives, such as penetration enhancers, with an organic solvent (e.g.; ethyl acetate, isopropanol, methanol, acetone, 2-butanone, ethanol, toluene, alkanes, and mixtures thereof) to provide a coating composition. The mixture is shaken or stirred until a homogeneous coating composition is obtained. The resulting composition is then applied to a release liner using conventional coating methods (e.g., knife coating or extrusion die coating) to provide a predetermined uniform thickness of coating composition. Non-continuous or discontinuous coatings may be prepared using methods such as stripe coating, screen printing, and ink jet printing.

Suitable release liners include conventional release liners comprising a known sheet material such as a polyester web, a polyethylene web, a polystyrene web, or a polyethylene-coated paper coated with a suitable fluoropolymer or silicone based coating. The release liner that has been coated with the composition is then dried and laminated onto a barrier layer using conventional methods. An optional tie layer may be used to connect the skin-contacting component with the barrier layer. Alternatively, the skin-contacting component compositions may be directly coated onto the barrier layer and subsequently dried and laminated to a release liner.

When the adverse agent or reservoir component comprises a pressure-sensitive adhesive or similar polymeric material or matrix, then the adverse agent or reservoir component compositions of the invention can be prepared using methods similar to those for preparing the active agent or skin-contacting component, with the exception that an antagonist is used in place of the active agent to prepare the coating composition. Alternatively, the adverse agent or reservoir component can comprise a porous medium, such as a porous or microporous film. The antagonist can be dissolved in an impregnating solvent and the porous or microporous film is soaked in the solvent for a sufficient period of time to allow the antagonist to penetrate the pores of the film. The solvent is then dried leaving the antagonist dispersed throughout the film. The adverse agent component is laminated to the barrier side of the barrier/skin-contacting multilaminate, optionally using heat or an additional tie layer to ensure adequate contact between the reservoir component and barrier.

A backing is laminated to the surface of the adverse agent or reservoir component opposed to the barrier, optionally using heat or an additional tie layer to ensure adequate contact between the reservoir component and backing. One skilled in the art will appreciate that it may be preferred to vary the order of lamination steps depending on the types and thickness of the layers comprising the dosage form.

The transdermal dosage forms of the invention can be made in the form of an article such as a tape, a patch, a sheet, a dressing or any other form known to those skilled in the art. Generally, the dosage form will be in the form of a patch of a size suitable to deliver a preselected amount of active agent through the skin.

Generally, the dosage form will have a surface area greater than 1 cm², preferably greater than 5 cm². Generally, the dosage form will have a surface area of less than 100 cm², preferably less than 40 cm².

Dosage forms of the present invention typically comprise a release liner that covers and protects the skin-contacting surface prior to use by a patient. Suitable release liners include conventional release liners comprising a known sheet material such as a polyester web, a polyethylene web, a polypropylene web, or a polyethylene-coated paper coated with a suitable fluoropolymer or silicone based coating. Dosage forms of the present invention are typically packaged individually in a foil-lined pouch for storage. Dosage forms of the present invention may alternatively be provided in a rolled or stacked form suitable for use with a dispensing apparatus.

In another embodiment, the present invention comprises a method of transdermal delivery of a drug from an abuse-resistant dosage form comprising the following steps. A step of providing a dosage form comprising an active agent component comprising a polymeric material and an active agent dispersed in the polymeric material, wherein the active agent component has skin-contacting surface, an adverse agent component comprising an antagonist to the active agent and a discontinuous barrier impermeable to diffusion of active agent and adverse agent, the barrier interposed between the active agent component and the adverse agent component. A step of providing at least one channel in the dosage form passing through the active agent component which substantially provides open fluid communication between the skin-contacting surface and the adverse agent component. A step of applying the dosage form to an external part of the human body for a period sufficient to achieve the desired therapeutic result. In one embodiment, the step of applying the dosage form to an external part of the human body follows the other two steps of providing the dosage form and providing at least one channel in the dosage form. Surface extraction with a solvent of such a dosage form will allow solvent to be in fluid communication with the adverse agent component. Thus surface extraction from such a dosage form will release a mixture of active agent and adverse agent. It is advantageous that such a channel be present in the dosage form prior to use by a patient, since this will act to prevent or discourage abuse or misuse of unused dosage forms.

### Examples

### In Vitro Skin Permeation Test Method

The skin permeation data given in the examples below was obtained using the following test method. A 5.0 cm² transdermal patch was die-cut from the center of a 10.0 cm² overlay patch (5.0 cm² active area) for use as the test sample. The release liner was removed, and the patch was applied to human cadaver skin and pressed to cause uniform contact with the skin. The resulting patch/skin laminate was placed patch side up across the orifice of the lower portion of a vertical diffusion cell. The diffusion cell was assembled and the lower portion filled with 25 mL of warm (32°C) receptor fluid (0.1 M phosphate buffer, pH 6.8) so that the receptor fluid contacted the skin. The sampling port was covered except when in use.

The cells were maintained at 32 ± 2°C throughout the course of the experiment. The receptor fluid was stirred by means of a magnetic stirrer throughout the experiment to assure a uniform sample and a reduced diffusion barrier on the dermal side of the skin. The entire volume of receptor fluid was withdrawn at specified time intervals and immediately replaced with fresh fluid. The withdrawn fluid was filtered through a 0.45 µm filter. The last 1-2 mL were then analyzed for fentanyl using conventional high performance liquid chromatography methods (Column: Zorbax SB AQ, 50 x 4.6 mm, 5 µm particle size; Mobile phase: 3-20% isopropanol in 22 mM phosphate buffer; Flow Rate: 1.5 mL/min; Detector: uv at 230 nm; Injection Volume: 10 µL; Run time: 6 minutes). The cumulative amount of fentanyl penetrating through the skin was calculated and reported as µg/cm². Unless noted, the results are reported as the average of 8 replicates.

### Extraction Method

The test samples were 3.5 cm² transdermal patches. The extraction solution was chosen from one of the following solutions: buffered saline (PBS, M phosphate buffer for pH 6.5, 0.5 M sodium chloride); diethyl ether (reagent grade with BHT preservative); deionized (DI) water; ethanol (USP, absolute); ethyl acetate (HPLC grade).

A 15 mL extraction solution was added into a 40 mL vial. The skin-adhesive side of the patch was applied to the rim of the vial, such that the patch completely covered the opening of the vial. A screw-on, teflon septum cap was placed over the patch to seal the vial. The sealed vial was stored in an upright position for not more than one hour prior to shaking.

The vial was shaken on a shaker table (IKA Labortechnik 501 Digital Shaker) set to 250 rpm. At fixed time intervals of 5, 15, and 30 minutes 0.5 mL aliquots were removed through the septum using a syringe. Each aliquot was placed into a 1 mL vial. If the extraction solvent was ethyl acetate or ether, then it was evaporated to dryness. Methanol (0.5 mL, HPLC grade) was added to the sample, mixed, and assayed for active drug substance by reverse-phase HPLC. If the extraction solvent was water or ethanol, then the sample was assayed directly for active and adverse agents by reverse-phase HPLC.

### Mechanical Separation Method

The test samples were 10.0 cm² overlay transdermal patches (active area 5.0 cm²). Ten individuals tested a single patch of each type. The testers were given diagrams indicating the individual layers of the patch. The testers were also provided with a scalpel, tweezers, and adhesive tape to use as tools. Each tester was given a one-hour time period and instructed to mechanically separate the patch in an attempt to separate the fentanyl from the naltrexone. Separated material believed to contain fentanyl and to be free of naltrexone was placed into a 40 mL vial, extracted with approximately 5 mL of methanol, and tested by HPLC for both fentanyl and naltrexone content. The results are reported as the average amount of fentanyl recovered from each patch, the average amount of naltrexone recovered from each patch, and the ratio of fentanyl to naltrexone recovered.

### Copolymer A. Preparation of Isooctyl Acrylate/2-Hydroxyethyl acrylate/Elvacite™ 1010 Copolymer Solution

A master batch was prepared by combining isooctyl acrylate (714.00 g), 2-hydroxyethyl acrylate (523.00g), polymethylmethacrylate macromonomer (52.00g) of ELVACITE^{™} 1010 available from ICI Acrylics), 2,2'-azobis(2-methylbutyronitrile) (2.60 g), ethyl acetate (1245.50g) and isopropanol (45.50 g). The resulting solution was divided in equal portions and placed into six 1 quart (0.95 L) amber glass bottles. The bottles were purged for 2 minutes with nitrogen at a flow rate of 1 L per minute. The bottles were sealed and placed in a rotating water bath at 57°C for 24 hours. At 24 hours the bottles were removed from the rotating water bath, unsealed, diluted with 76 g methanol per bottle, mixed until homogenous, and recombined into a 1 gallon (3.8 L) glass jar. The percent solids of the resultant copolymer was 40.5%. The inherent viscosity (of a 0.15 g/dL solution of polymer in ethyl acetate measured at 27°C) was 0.77 dL/g.

### Copolymer B. Preparation of 2-Ethylhexyl acrylate/Dimethylaminoethyl acrylate methyl chloride quaternary/Methoxy polyethylene glycol 400 acrylate Copolymer Solution

A master batch was prepared by combining 2-ethylhexyl acrylate (234 g), dimethylaminoethyl acrylate methyl chloride quaternary (90 g), methoxy polyethylene glycol 400 acrylate (54 g), methanol (200.84 g) and acetone (221.14 g). The resulting solution was divided in equal portions and placed into two 1 quart (0.95 L) amber glass bottles. The bottles were purged for 2 minutes with nitrogen at a flow rate of 1 L per minute. The bottles were sealed and placed in a rotating water bath at 57 °C for 24 hours. At 24 hours the bottles were removed from the rotating water bath and cooled. Methanol (50 g) and acetone (50 g) were added to each bottle and mixed until homogeneous. The resulting solutions were then treated with radical initiators for an additional 6 hours at 57 °C to reduce the amount of remaining residual monomers. The resulting copolymer solutions in the two bottles were recombined into a 1 gallon (3.8 L) glass jar. The percent solids of the resultant copolymer was 36.3 %. The Brookfield viscosity was 835 centipoise.

### Copolymer C. Polyurea Copolymer Solution

Polyoxypropylenediamine (198.75 g, Jeffamine® D2000, Huntsman Co., Houston, TX), Polyoxyalkyleneamine (66.25 g, Jeffamine® XTJ 502, Huntsman Co., Houston, TX), 2-methyl-1,5-pentanediamine (0.44 g), and 2-propanol (301.14 g) were added to a 1 quart (0.95 L) jar and mixed until homogeneous. Dicyclohexylmethane-4,4'-diisocyanate (35.70 g) was then added to the jar with a 2-propanol wash and mixed for 16 hours to prepare a polymer solution.

The resulting solution was knife coated at a wet thickness of 22 mil (559 µm) onto an in-process silicone coated release liner and dried for 4 minutes at 110 °F (43 °C), for 4 minutes at 185 °F (85 °C), and for 4 minutes at 200 °F (93.3 °C). The dried copolymer (161.8 g) was then added to acetone (242.7 g) to prepare a 40.1% solids solution.

### Porous Polyethylene Film

A porous polyethylene film was produced according to the method described in U.S. Patent No. 4, 539, 256 (Shipman). Briefly, molten high density polyethylene (Finathene® 7208, Atofina Petrochemicals, Houston, Texas) was mixed with a USP-grade mineral oil (Chevron Superla® White Oil 31, Chevron Products Co., San Ramon, CA) and extruded onto a water-cooled wheel whereupon an oil-filled membrane was formed. The oil was then removed via washing with a solvent, followed by biaxial stretching to form a 5 mil (127 µm) thick porous film. The porous film was 74% porous and had a 250 nm bubble point pore size. The surface of the film that contacts the water-cooled wheel is referred to as the "wheel" side. The porous film was rendered hydrophilic via three sequential plasma etching treatments in a silane and oxygen plasma.

### Example 1

A transdermal dosage form according to FIG. 7a, b was prepared as follows.

Fentanyl (19.5 g) was added to methanol (23.5 g) and mixed until all of the fentanyl was dissolved. To this solution, copolymer (251.6 g of the solution of isooctyl acrylate/2-hydroxyethyl acrylate/Elvacite™ 1010 from Copolymer A above) was added and mixed until a uniform coating formulation was obtained. The coating formulation was knife coated onto a silicone release liner. The coated liner was oven dried for 4 minutes at 110 °F (43 °C), for 4 minutes at 185 °F (85 °C), and for 4 minutes at 200 °F (93.3 °C). The resulting dried coating weight was 12.6 mg/cm². The resulting coating contained 16.0 percent fentanyl. The coated liner was laminated onto an in-process silicone release liner for temporary storage of the dried fentanyl-copolymer coating.

A naltrexone solution with a concentration of 0.2658 g/mL was prepared in tetrahydrofuran. The naltrexone solution was coated onto the wheel side of the porous polyethylene film described above and dried for 20 minutes at 125 °F (51.7 °C). The resulting film had a naltrexone concentration of 3.11 mg/cm².

A fluoropolymer coating of 30% (w/w) tetrafluoroethylene-hexafluoropropylene-vinylidene fluorite terpolymer (THV 220, Dyneon, Oakdale, MN) in acetone was then applied to the wheel side of the naltrexone impregnated film using a #5 Mayer rod. The resulting film had a nominal dried thickness of approximately 0.15 mil (4 µm).

The in-process silicone release liner from the fentanyl coating was removed and the dried coating was laminated to the fluoropolymer coating on the porous polyethylene film to form a multilaminate construction.

The resulting multilaminate construction was converted into 3.5 cm² and 5.0 cm² patches. Nine approximately evenly spaced holes, each with an area of 0.013 cm², were punched through the full thickness of each 3.5 cm² patch. A Tegaderm^{™} dressing was adhered to the side of the porous polyethylene film opposed to the fluoropolymer coating of each 3.5 cm² patch and trimmed to an area of 3.5 cm². Fifteen approximately evenly spaced holes, each with an area of 0.013 cm², were punched through the full thickness of each 5.0 cm² patch. A Tegaderm^{™} dressing was adhered to the side of the porous polyethylene film opposed to the fluoropolymer coating of each 5.0 cm² patch as an overlay backing and overlay PSA as shown in FIG. 14. The Tegaderm^{™} dressing was trimmed so that it extended 5 mm around the edges of the 5.0 cm² patch.

Permeation of both fentanyl and naltrexone through human cadaver skin was determined using the test method described above. The results are shown in Tables 1 and 2. Solvent extraction was determined using the test method described above. The results are shown in Table 3. Mechanical separation testing was performed using the test method described above. The results are shown in Table 4.

| Table 1 | | | | | | | | | | | |
|---|---|---|---|---|---|---|---|---|---|---|---|
| Human Cadaver Skin Permeation | | | | | | | | | | | |
| Example Number | Average Flux Fentanyl (µg/cm²/hr) | | | | | | | | | | |
| | 4 hr | 8 hr | 12 hr | 24 hr | 36 hr | 48 hr | 72 hr | 96 hr | 120 hr | 144 hr | 168 hr |
| 1 | 0.0 | 0.4 | 0.9 | 1.2 | 1.3 | 1.1 | 1.1 | 1.0 | 1.0 | 0.9 | 0.9 |
| 2 | 0.0 | 0.2 | 0.5 | 0.9 | 1.1 | 1.0 | 1.0 | 0.9 | 1.0 | 0.9 | 0.9 |
| 3 | 0.7 | 2.4 | 3.5 | 3.8 | 4.3 | 4.3 | 4.4 | 4.4 | 3.6 | 3.5 | 3.2 |

| Table 2 | | | | | | | | | | | |
|---|---|---|---|---|---|---|---|---|---|---|---|
| Human Cadaver Skin Permeation | | | | | | | | | | | |
| Example Number | Average Flux Naltrexone (µg/cm²/hr) | | | | | | | | | | |
| | 4 hr | 8 hr | 12 hr | 24 hr | 36 hr | 48 hr | 72 hr | 96 hr | 120 hr | 144 hr | 168 hr |
| 1 | 0.52 | 0.00 | 0.01 | 0.00 | 0.09 | 0.01 | 0.05 | 0.14 | 0.01 | 0.02 | 0.03 |
| 2 | 0.21 | 0.00 | 0.00 | 0.00 | 0.00 | 0.00 | 0.01 | 0.05 | 0.00 | 0.00 | 0.00 |
| 3 | 0.09 | 0.00 | 0.00 | 0.00 | 0.02 | 0.01 | 0.01 | 0.00 | 0.01 | 0.01 | 0.01 |

| Table 3 | | | | | | | | | | |
|---|---|---|---|---|---|---|---|---|---|---|
| Solvent Extraction | | | | | | | | | | |
| Example Number | | Ratio Fentanyl/Naltrexone | | | Naltrexone Extracted [mg] | | | Fentanyl Extracted [mg] | | |
| | Solvent | 5 min | 15 min | 30 min | 5 min | 15 min | 30 min | 5 min | 15 min | 30 min |
| 1 | DI Water | 53.8 | 70.9 | 57.6 | 0.0 | 0.0 | 0.0 | 0.1 | 0.2 | 0.3 |
| 1 | PBS | 37.0 | 32.0 | 14.1 | 0.1 | 0.2 | 0.3 | 0.2 | 0.3 | 0.4 |
| 1 | Ethanol | 171 | 50.8 | 22.7 | 0.0 | 0.1 | 0.3 | 3.6 | 4.8 | 5.2 |
| 1 | Ethyl Acetate | 1.1 | 0.8 | 0.7 | 4.4 | 6.5 | 7.6 | 4.7 | 4.9 | 5.1 |
| 1 | Diethyl Ether | 4.9 | 3.9 | 2.1 | 0.9 | 2.0 | 3.3 | 4.0 | 4.3 | 4.2 |
| 2 | DI Water | 1.0 | 1.0 | 0.9 | 0.1 | 0.2 | 0.4 | 0.1 | 0.2 | 0.3 |
| 2 | PBS | 0.8 | 0.9 | 1.0 | 0.2 | 0.4 | 0.4 | 0.2 | 0.3 | 0.4 |
| 2 | Ethanol | 3.4 | 2.2 | 1.4 | 1.1 | 2.2 | 3.8 | 3.8 | 4.8 | 5.1 |
| 2 | Ethyl Acetate | 3.7 | 2.4 | 1.9 | 1.7 | 2.7 | 3.4 | 5.2 | 5.7 | 5.8 |
| 2 | Diethyl Ether | 4.5 | 2.3 | 1.5 | 1.0 | 2.1 | 3.2 | 3.9 | 4.4 | 4.6 |
| 3 | DI Water | 1.2 | 1.8 | 1.2 | 0.2 | 0.4 | 0.6 | 0.1 | 0.3 | 0.4 |
| 3 | PBS | 2.0 | 1.5 | 1.2 | 0.1 | 0.2 | 0.4 | 0.2 | 0.3 | 0.4 |
| 3 | Ethanol | 3.5 | 2.1 | 1.2 | 1.2 | 2.5 | 4.4 | 4.0 | 5.2 | 5.2 |
| 3 | Ethyl Acetate | 1.7 | 1.2 | 1.0 | 3.6 | 5.2 | 4.6 | 5.3 | 5.4 | 4.4 |
| 3 | Diethyl Ether | 2.5 | 1.2 | 0.8 | 2.2 | 3.9 | 5.8 | 3.9 | 4.5 | 4.8 |

| Table 4 | | | |
|---|---|---|---|
| Mechanical Separation | | | |
| Example Number | Fentanyl [mg/patch] | Naltrexone [mg/patch] | Ratio Fentanyl/Naltrexone |
| 1 | 1.2 | 0.7 | 1.7 |
| 2 | 1.3 | 1.4 | 0.9 |
| 3 | 0.9 | 1.4 | 0.6 |

### Example 2

A transdermal dosage form according to FIG. 6a, b was prepared as follows.

An antagonist reservoir was prepared as follows. Naltrexone (13.55g) was added to copolymer (149.4 g of the solution of 2-ethylhexyl acrylate/dimethylaminoethyl acrylate methyl chloride quaternary/methoxy polyethylene glycol 400 acrylate from copolymer B above) and mixed until homogeneous. The coating formulation was knife coated onto a silicone release liner. The coated liner was oven dried for 4 minutes at 110 °F (43 °C), for 4 minutes at 185 °F (85 °C), and for 4 minutes at 200 °F (93.3 °C). The resulting dried coating weight was 14.2 mg/cm².

A dried fentanyl-copolymer coating was prepared as described in Example 1. The in-process silicone release liner was removed from the dried fentanyl-copolymer coating, and the dried fentanyl-copolymer coating was laminated to the ethylene vinyl acetate side of a 2.0 mil (51 µm) thick laminate film of polyethylene terephthalate (PET) and ethylene vinyl acetate (Scotchpak^{™} 9732, 3M, St. Paul, MN).

The resulting multilaminate construction was converted into 3.5 cm² and 5.0 cm² parts. Nine approximately evenly spaced holes, each with an area of 0.013 cm², were punched through the full thickness of each 3.5 cm² part. Fifteen approximately evenly spaced holes, each with an area of 0.013 cm², were punched through the full thickness of each 5.0 cm² part.

The PET side of each multilaminate 3.5 or 5.0 cm² part was laminated to the exposed surface of the dried naltrexone coating to form a multilaminate construction. The resulting multilaminate construction was converted into 3.5 or 5 cm² patches, respectively.

The in-process silicone release liner was removed from the dried naltrexone coating and a Tegaderm^{™} dressing was adhered to the dried naltrexone coating of each 3.5 cm² patch and trimmed to an area of 3.5 cm². The in-process silicone release liner was removed from the dried naltrexone coating and a Tegaderm^{™} dressing was adhered to the dried naltrexone coating of each 5.0 cm² patch as an overlay backing and overlay PSA as shown in FIG. 14. The Tegaderm^{™} dressing was trimmed so that it extended 5 mm around the edges of the 5.0 cm² patch.

Permeation of both fentanyl and naltrexone through human cadaver skin was determined using the test method described above. The results are shown in Tables 1 and 2. Solvent extraction was determined using the test method described above. The results are shown in Table 3. Mechanical separation testing was performed using the test method described above. The results are shown in Table 4.

### Example 3

A transdermal dosage form according to FIG. 6a, b was prepared as follows.

A dried naltrexone coating was prepared as follows. Naltrexone (14.00 g), acetone (35.1 g), tetrahydrofuran (13.1 g), and copolymer (140 g of a 40.1% solids solution in acetone of polyurea copolymer C above) were added together and mixed until homogeneous. The resulting composition was coated onto an in-process silicone coated release liner and dried for 4 minutes at 110 °F (43 °C), for 4 minutes at 185 °F (85 °C), and for 4 minutes at 200 °F (93.3 °C). The resulting dried coating contained 20.0 percent naltrexone. The resulting dried coating weight was approximately 15.7 mg/cm².

A dried fentanyl-copolymer coating was prepared as described in Example 1. The dried fentanyl-copolymer coating was laminated to the polyethylene terephthalate side of a 2.0 mil (51 µm) thick laminate film of polyethylene terephthalate and ethylene vinyl acetate (Scotchpak^{™} 9732, 3M, St. Paul, MN).

The resulting multilaminate construction was converted into 3.5 cm² and 5.0 cm² parts. Nine evenly spaced holes, each with an area of 0.013 cm², were punched through the full thickness of each 3.5 cm² part. Fifteen evenly spaced holes, each with an area of 0.013 cm², were punched through the full thickness of each 5.0 cm² part.

The ethylene vinyl acetate side of each multilaminate 3.5 or 5.0 cm² part was laminated to the exposed surface of the dried naltrexone coating to form a multilaminate construction. The resulting multilaminate construction was converted into 3.5 or 5 cm² patches, respectively.

The in-process silicone release liner was removed from the dried naltrexone coating and a Tegaderm^{™} dressing was adhered to the dried naltrexone coating of each 3.5 cm² patch and trimmed to an area of 3.5 cm². The in-process silicone release liner was removed from the dried naltrexone coating and a Tegaderm^{™} dressing was adhered to the dried naltrexone coating of each 5.0 cm² patch as an overlay backing and overlay PSA as shown in FIG. 14. The Tegaderm^{™} dressing was trimmed so that it extended 5 mm around the edges of the 5.0 cm² patch.

Permeation of both fentanyl and naltrexone through human cadaver skin was determined using the test method described above. The results are shown in Tables 1 and 2. Solvent extraction was determined using the test method described above. The results are shown in Table 3. Mechanical separation testing was performed using the test method described above. The results are shown in Table 4.

### Example 4

A transdermal drug delivery device according to FIG. 1a, b was prepared as follows.

Fentanyl (19.5 g) was added to methanol (23.5 g) and mixed until all of the fentanyl was dissolved. To this solution, copolymer (251.6 g of the solution of isooctyl acrylate/2-hydroxyethyl acrylate/Elvacite™ 1010 (57/39/4) from Copolymer A above) was added and mixed until a uniform coating formulation was obtained. The coating formulation was knife coated using a slotted knife onto a silicone release liner using coating dams to create stripes of adhesive. The coated stripes were approximately 5 mm wide and separated with uncoated areas that were approximately 1.5 mm wide. The coated liner was oven dried for 4 minutes at 110 °F (43 °C), for 4 minutes at 185 °F (85 °C), and for 4 minutes at 200 °F (93.3 °C). The resulting dried coating weight was approximately 10.5 mg/cm2 in the coated areas. The resulting coating contained 16.0 percent fentanyl. The coated liner was laminated onto an in-process silicone release liner for temporary storage of the dried fentanyl-copolymer coating.

A polyvinylalcohol (PVA) film was prepared from the following stock solutions. Stock solution A was prepared by adding 50.0g of polyvinyl alcohol (87-89% hydrolyzed, 124,000-186,000 molecular weight) into a beaker containing 450.0 g of deionized water. The mixture was warmed on a hot plate and stirred constantly until the solution was homogeneous (approximately 30 minutes). Stock solution B was prepared by adding 4.0 g of polyacrylic acid (molecular weight 1,250,000) to 196.3g of deionized water and stirring until homogenous. Stock solution C was prepared by adding 22.9 g of glyceryl monolaurate to 37.7 g of isopropyl alcohol and 16.0 g of deionized water. The mixture was heated in an oven at 140 °F (60 °C) for 30 minutes to dissolve the glyceryl monolaurate .Stock solution A (305.0 g) was mixed with stock solution B (99.8 g) until homogeneous. To this solution, stock solution C (58.3 g) was added and mixed until homogeneous. The resulting solution had a percent solids of 11% and a composition of 61:4:35 polyvinyl alcohol:polyacrylic acid:glyceryl monolaurate.

This solution was knife coated at a wet thickness of 10 mil (254 µm) onto a silicone coated release liner. The coated liner was oven dried for 4 minutes at 185 °F (85 °C) and 6 minutes at 225 °F (107 °C) to form a PVA film. The resulting dried coating weight was approximately 2 mg/cm². The in-process silicone release liner from the dried fentanyl-copolymer coating was removed and the dried coating was laminated to the PVA film to form a PVA-fentanyl coating laminate.

An antagonist reservoir component was prepared as described in Example 2. The resulting dried coating contained 20.0 percent naltrexone. The resulting dried coating weight was approximately 14.2 mg/cm².

The exposed surface of the dried naltrexone coating was laminated to the exposed PVA surface of the PVA-fentanyl coating laminate to form a multilaminate construction.

The resulting multilaminate construction was converted into 3.5 cm² and 5.0 cm² patches. The in-process silicone release liner was removed from the dried naltrexone coating and a Tegaderm^{™} dressing was adhered to the dried naltrexone coating of each 3.5cm² patch and trimmed to an area of 3.5 cm². The in-process silicone release liner was removed from the dried naltrexone coating and a Tegaderm^{™} dressing was adhered to the dried naltrexone coating of each 5.0 cm² patch as an overlay backing and overlay PSA as shown in FIG. 14. The Tegaderm dressing was trimmed so that it extended 5 mm around the edges of the of the 5.0 cm² patch.

Solvent extraction was determined using the test method described above. The results are shown in Table 5. Mechanical separation testing was performed using the test method described above. The results are shown in Table 6.

| Table 5 | | | | | | | | | | |
|---|---|---|---|---|---|---|---|---|---|---|
| Solvent Extraction | | | | | | | | | | |
| Example Number | | Ratio | Fentanyl/Naltrexone | | Naltrexone Extracted [mg] | | | Fentanyl Extracted [mg] | | |
| | Solvent | 5 min | 15 min | 30 min | 5 min | 15min | 30 min | 5 min | 15 min | 30 min |
| 4 | DI Water | 42.3 | 16.3 | 7.7 | 0.0 | 0.1 | 0.2 | 0.1 | 0.2 | 0.3 |
| 4 | PBS | 2.2 | 1.4 | 1.0 | 0.1 | 0.2 | 0.4 | 0.2 | 0.3 | 0.4 |
| 4 | Ethanol | 1385 | 84.4 | 34.4 | 0.0 | 0.1 | 0.1 | 2.9 | 4.0 | 4.2 |
| 4 | Ethyl Acetate | 136 | 62.7 | 28.1 | 0.0 | 0.1 | 0.2 | 4.1 | 4.4 | 4.5 |
| 4 | Diethyl Ether | 377 | 131 | 55.8 | 0.0 | 0.0 | 0.1 | 3.3 | 3.8 | 3.9 |
| 5 | DI Water | 14.0 | 10.6 | 7.5 | 0.1 | 0.2 | 0.4 | 0.1 | 0.3 | 0.6 |
| 5 | PBS | 24.4 | 27.8 | 14.9 | 0.1 | 0.3 | 0.6 | 0.7 | 1.3 | 1.8 |
| 5 | Ethanol | 2254 | 227 | 101 | 0.0 | 0.0 | 0.1 | 5.0 | 7.3 | 7.6 |
| 5 | Ethyl Acetate | 68.7 | 22.7 | 10.4 | 0.1 | 0.3 | 0.8 | 5.5 | 5.7 | 5.9 |
| 5 | Diethyl Ether | 203 | 120 | 67.3 | 0.0 | 0.0 | 0.1 | 4.5 | 4.7 | 4.8 |
| 6 | DI Water | 4.5 | 1.3 | 0.6 | 0.0 | 0.1 | 0.5 | 0.1 | 0.2 | 0.3 |
| 6 | PBS | 2.2 | 1.6 | 0.9 | 0.1 | 0.2 | 0.4 | 0.2 | 0.3 | 0.4 |
| 6 | Ethanol | 152 | 35.6 | 9.1 | 0.0 | 0.2 | 0.7 | 3.1 | 4.4 | 4.7 |
| 6 | Ethyl Acetate | 68.5 | 11.4 | 3.6 | 0.1 | 0.4 | 1.2 | 4.1 | 4.4 | 4.5 |
| 6 | Diethyl Ether | 19.7 | 12.3 | 7.5 | 0.2 | 0.3 | 0.5 | 3.3 | 3.7 | 3.9 |
| 7 | DI Water | 4.0 | 0.7 | 0.5 | 0.0 | 0.4 | 0.8 | 0.1 | 0.3 | 0.4 |
| 7 | PBS | 4.6 | 2.5 | 1.5 | 0.2 | 0.5 | 1.1 | 0.6 | 1.1 | 1.5 |
| 7 | Ethanol | 320 | 67.4 | 13.3 | 0.0 | 0.1 | 0.6 | 4.4 | 5.8 | 6.1 |
| 7 | Ethyl Acetate | 71.5 | 22.2 | 4.5 | 0.1 | 0.2 | 1.2 | 5.1 | 5.1 | 5.3 |
| 7 | Diethyl Ether | 25.8 | 18.0 | 11.6 | 0.2 | 0.3 | 0.4 | 4.3 | 4.2 | 4.6 |
| 8 | DI Water | 1.7 | 1.5 | 1.2 | 0.1 | | 0.3 | 0.1 | 0.2 | 0.2 |
| 8 | PBS | 3.6 | 1.7 | 1.1 | 0.2 | 0.4 | 1.1 | 0.1 | 0.3 | 0.4 |
| 8 | Ethanol | 9.9 | 3.5 | 1.5 | 0.4 | 1.9 | 3.9 | 2.4 | 4.1 | 4.3 |
| 8 | Ethyl Acetate | NA | NA | NA | NA | NA | NA | NA | NA | NA |
| 8 | Diethyl Ether | 3.4 | 1.5 | 0.9 | 1.6 | 4.1 | 6.5 | 3.6 | 4.1 | 4.5 |
| 9 | DI Water | 0.7 | 0.7 | 0.7 | 0.2 | 0.4 | 0.7 | 0.1 | 0.3 | 0.5 |
| 9 | PBS | 0.6 | 0.3 | 0.3 | 0.8 | 3.6 | 5.8 | 0.4 | 1.0 | 1.6 |
| 9 | Ethanol | 3.9 | 1.3 | 1.0 | 1.3 | 4.4 | 5.8 | 4.1 | 5.4 | 5.7 |
| 9 | Ethyl Acetate | 3.8 | 2.0 | 1.3 | 1.4 | 2.9 | 4.4 | 5.2 | 5.6 | 5.6 |
| 9 | Diethyl Ether | 1.5 | 1.0 | 0.8 | 2.5 | 4.8 | 6.3 | 3.3 | 4.3 | 4.7 |

| Table 6 | | | |
|---|---|---|---|
| Mechanical Separation | | | |
| Example Number | Fentanyl [mg/patch] | Naltrexone [mg/patch] | Ratio Fentanyl/Naltrexone |
| 4 | 0.8 | 1.2 | 0.7 |
| 5 | 1.3 | 0.4 | 3.6 |
| 6 | 1.7 | 1.5 | 1.1 |
| 7 | 1.6 | 1.3 | 1.2 |
| 8 | NA | NA | NA |
| 9 | 1.2 | 0.5 | 2.5 |

### Example 5

A transdermal dosage form according to FIG. 1a, b was prepared as follows.

Fentanyl (10.3 g) was added to methanol (12.4 g) and mixed until all of the fentanyl was dissolved. To this solution, methyl laurate (15.0 g) and copolymer (85.6 g of the solution of isooctyl acrylate/2-hydroxyethyl acrylate/Elvacite™ 1010 from Copolymer A above) were added and mixed until a uniform coating formulation was obtained. The coating formulation was knife coated using a slotted knife onto a silicone release liner using coating dams to create stripes of adhesive. The coated stripes were approximately 5 mm wide and separated with uncoated areas that were approximately 1.5 mm wide. The coated liner was oven dried for 4 minutes at 110 °F (43 °C), for 4 minutes at 185 °F (85 °C), and for 4 minutes at 200 °F (93.3 °C). The resulting dried coating weight was approximately 14.1 mg/cm² in the coated areas. The resulting coating contained 17.1 % fentanyl. The coated liner was laminated onto an in-process silicone release liner for temporary storage of the dried fentanyl-methyl laurate-copolymer coating.

A PVA film was prepared as described in example 4. The in-process silicone release liner from the dried fentanyl-copolymer coating was removed and the dried coating was laminated to the PVA film to form a PVA-fentanyl coating laminate.

An antagonist reservoir component was prepared as described in Example 2. The resulting dried coating contained 20.0 percent naltrexone. The resulting dried coating weight was approximately 14.2 mg/cm².

The exposed surface of the dried naltrexone coating was laminated to the exposed PVA surface of the PVA-fentanyl coating laminate to form a multilaminate construction.

The resulting multilaminate construction was converted into 3.5 cm² and 5.0 cm² patches. The in-process silicone release liner was removed from the dried naltrexone coating and a Tegaderm^{™} dressing was adhered to the dried naltrexone coating of each 3.5cm² patch and trimmed to an area of 3.5 cm². The in-process silicone release liner was removed from the dried naltrexone coating and a Tegaderm^{™} dressing was adhered to the dried naltrexone coating of each 5.0 cm² patch as an overlay backing and overlay PSA as shown in FIG. 14. The Tegaderm dressing was trimmed so that it extended 5 mm around the edges of the of the 5.0 cm² patch.

Solvent extraction was determined using the test method described above. The results are shown in Table 5. Mechanical separation testing was performed using the test method described above. The results are shown in Table 6.

### Example 6

A transdermal dosage form according to FIG. 1a, b was prepared as follows.

A PVA-fentanyl coating laminate was prepared as described in Example 4. A dried naltrexone coating was prepared as described in Example 3. The exposed surface of the dried naltrexone coating was laminated to the exposed PVA surface of the PVA-fentanyl coating laminate to form a multilaminate construction.

The resulting multilaminate construction was converted into 3.5 cm² and 5.0 cm² patches. The in-process silicone release liner was removed from the dried naltrexone coating and a Tegaderm^{™} dressing was adhered to the dried naltrexone coating of each 3.5cm² patch and trimmed to an area of 3.5 cm². The in-process silicone release liner was removed from the dried naltrexone coating and a Tegaderm^{™} dressing was adhered to the dried naltrexone coating of each 5.0 cm² patch as an overlay backing and overlay PSA as shown in FIG. 14. The Tegaderm dressing was trimmed so that it extended 5 mm around the edges of the of the 5.0 cm² patch.

Permeation through human cadaver skin was determined using the test method described above. The results are shown in Table 1. Solvent extraction was determined using the test method described above. The results are shown in Table 2.

### Example 7

A transdermal dosage form according to FIG. 1a, b was prepared as follows.

A PVA-fentanyl coating laminate was prepared as described in Example 5. A dried naltrexone coating was prepared as described in Example 3. The exposed surface of the dried naltrexone coating was laminated to the exposed PVA surface of the PVA-fentanyl coating laminate to form a multilaminate construction.

The resulting multilaminate construction was converted into 3.5 cm² and 5.0 cm² patches. The in-process silicone release liner was removed from the dried naltrexone coating and a Tegaderm^{™} dressing was adhered to the dried naltrexone coating of each 3.5cm² patch and trimmed to an area of 3.5 cm². The in-process silicone release liner was removed from the dried naltrexone coating and a Tegaderm^{™} dressing was adhered to the dried naltrexone coating of each 5.0 cm² patch as an overlay backing and overlay PSA as shown in FIG. 14. The Tegaderm dressing was trimmed so that it extended 5 mm around the edges of the of the 5.0 cm² patch.

Solvent extraction was determined using the test method described above. The results are shown in Table 5. Mechanical separation testing was performed using the test method described above. The results are shown in Table 6.

### Example 8

A transdermal dosage form according to FIG. 1a, b was prepared as follows.

A naltrexone solution with a concentration of 30.0 % (w/w) was prepared in tetrahydrofuran. The naltrexone solution was coated onto the wheel side of the porous polyethylene film described above and dried for 12 minutes at 125 °F (51.7 °C). The resulting film had a naltrexone concentration of 3.2 mg/cm².

A solution having 11% percent solids and a composition of 61:4:35 polyvinyl alcohol:polyacrylic acid:glyceryl monolaurate was prepared as described in Example 4. This solution was coated with a #12 Mayer rod onto the naltrexone-loaded porous polyethylene film described above and oven dried for 10 minutes at 140 °F (60 °C) to prepare a PVA-porous polyethylene multilaminate.

A dried fentanyl-copolymer coating was prepared as described in Example 4. The in-process silicone release liner from the dried fentanyl-copolymer coating was removed and the dried coating was laminated to the PVA film to form a fentanyl-PVA-porous polyethylene multilaminate.

The resulting multilaminate construction was converted into 3.5 cm² and 5.0 cm² patches. A Tegaderm^{™} dressing was adhered to the porous polyethylene film of each 3.5cm² patch and trimmed to an area of 3.5 cm². A Tegaderm^{™} dressing was adhered to the porous polyethylene film of each 5.0 cm² patch as an overlay backing and overlay PSA as shown in FIG. 14. The Tegaderm dressing was trimmed so that it extended 5 mm around the edges of the of the 5.0 cm² patch

Solvent extraction was determined using the test method described above. The results are shown in Table 5.

### Example 9

A transdermal dosage form according to FIG. 1a, b was prepared as follows.

A dried fentanyl-methyl laurate-copolymer coating was prepared as described in Example 5.

A naltrexone solution with a concentration of 29.9% (w/w) was prepared in tetrahydrofuran. The naltrexone solution was coated onto the wheel side of the porous polyethylene film described above and dried for 20 minutes at 125 °F (51.7 °C). The resulting film had a naltrexone concentration of 2.99 mg/cm².

A solution having 11% percent solids and a composition of 61:4:35 polyvinyl alcohol:polyacrylic acid:glyceryl monolaurate was prepared as described in Example 4. This solution was coated with a #12 Mayer rod onto the wheel side of the naltrexone-loaded porous polyethylene film described above and oven dried for 10 minutes at 140 °F (60 °C) to prepare a PVA-porous polyethylene multilaminate.

The in-process silicone release liner from the dried fentanyl-methyl laurate-copolymer coating was removed and the dried coating was laminated to the PVA film to form a fentanyl-PVA-porous polyethylene multilaminate.

The resulting multilaminate construction was converted into 3.5 cm² and 5.0 cm² patches. A Tegaderm^{™} dressing was adhered to the porous polyethylene film of each 3.5cm² patch and trimmed to an area of 3.5 cm². A Tegaderm^{™} dressing was adhered to the porous polyethylene film of each 5.0 cm² patch as an overlay backing and overlay PSA as shown in FIG. 14. The Tegaderm dressing was trimmed so that it extended 5 mm around the edges of the of the 5.0 cm² patch. Solvent extraction was determined using the test method described above. The results are shown in Table 5. Mechanical separation testing was performed using the test method described above. The results are shown in Table 6.

## Claims

1. A transdermal dosage form comprising:
an active agent component comprising an active agent dispersed in a polymeric material, wherein the active agent component has a proximal, skin-contacting surface and a distal surface, and at least one channel passing between the proximal and distal surfaces;
an adverse agent component comprising an antagonist to the active agent; and
a barrier interposed between the distal surface of the active agent component and the adverse agent component;
wherein the barrier is permeable to a solvent selected from the group consisting of water, ethanol, ether, and mixtures thereof and further wherein the barrier is impermeable to diffusion of active agent and adverse agent in the absence of said solvent.

2. A transdermal dosage form according to claim 1, wherein the barrier is soluble in a solvent selected from the group consisting of water, ethanol, ether, and mixtures thereof.

3. A transdermal dosage form according to claim 1, wherein the barrier is permeable to water, ethanol, and mixtures thereof.

4. A transdermal dosage form according to claim 1, wherein the dosage form comprises a plurality of channels passing between the proximal and distal surfaces of the active agent component.

5. A transdermal dosage form according to claim 4, wherein the channels comprise a material that is soluble in a solvent selected from the group consisting of water, ethanol, ether, and mixtures thereof.

6. A transdermal dosage form according to claim 4, wherein the channels comprise beads soluble in a solvent selected from the group consisting of water, ethanol, ether, and mixtures thereof.

7. A transdermal dosage form comprising:
an active agent component comprising a polymeric material and an active agent dispersed in the polymeric material, wherein the active agent component has skin-contacting surface;
an adverse agent component comprising an antagonist to the active agent; and
a discontinuous barrier impermeable to diffusion of active agent and adverse agent, the barrier being interposed between the active agent component and the adverse agent component;
wherein the dosage form comprises at least one channel passing between the skin-contacting surface and the adverse agent component.

8. A transdermal dosage form according to claim 1 or 7, wherein the channel comprises an air channel.

9. A transdermal dosage form according to claim 1 or 7, further comprising a backing, wherein the adverse agent component is interposed between the barrier and the backing.

10. A transdermal dosage form according to claim 1 or 7, wherein the antagonist comprises a narcotic antagonist.

11. A transdermal dosage form according to claim 1 or 7, wherein the adverse agent component comprises a pressure-sensitive adhesive.

12. A transdermal dosage form according to claim 1 or 7, wherein the active agent is fentanyl.

13. A transdermal dosage form according to claim 7, wherein the barrier comprises a film comprising apertures.

14. A transdermal dosage form according to claim 13, wherein the apertures in the barrier are aligned with apertures in the active agent component.

15. A transdermal dosage form according to claim 14, wherein the adverse agent component comprises apertures that are aligned with the apertures in the barrier.

16. A transdermal dosage form according to claim 7, wherein the adverse agent component comprises a porous film.

## Patentansprüche

1. Transdermale Dosierungsform, umfassend:
eine Wirkstoffkomponente, die einen Wirkstoff umfasst, der in einem Polymermaterial dispergiert ist, wobei die Wirkstoffkomponente eine proximale, die Haut berührende Oberfläche und eine distale Oberfläche sowie mindestens einen Kanal aufweist, der zwischen der proximalen und der distalen Oberfläche verläuft,
eine Gegenwirkstoffkomponente, die einen Antagonisten des Wirkstoffes umfasst, und
eine Barriere, die zwischen der distalen Oberfläche der Wirkstoffkomponente und der Gegenwirkstoffkomponente eingefügt ist,
wobei die Barriere für ein Lösemittel durchlässig ist, das aus der Gruppe ausgewählt ist, die aus Wasser, Ethanol, Ether und Mischungen daraus besteht, und wobei ferner die Barriere in Abwesenheit des Lösemittels undurchlässig für die Diffusion von Wirkstoff und Gegenwirkstoff ist.

2. Transdermale Dosierungsform nach Anspruch 1, wobei die Barriere in einem Lösemittel löslich ist, das aus der Gruppe ausgewählt ist, die aus Wasser, Ethanol, Ether und Mischungen daraus besteht.

3. Transdermale Dosierungsform nach Anspruch 1, wobei die Barriere für Wasser, Ethanol und Mischungen daraus durchlässig ist.

4. Transdermale Dosierungsform nach Anspruch 1, wobei die Dosierungsform mehrere Kanäle umfasst, die zwischen der proximalen und der distalen Oberfläche der Wirkstoffkomponente verlaufen.

5. Transdermale Dosierungsform nach Anspruch 4, wobei die Kanäle ein Material umfassen, das in einem Lösemittel löslich ist, das aus der Gruppe ausgewählt ist, die aus Wasser, Ethanol, Ether und Mischungen daraus besteht.

6. Transdermale Dosierungsform nach Anspruch 4, wobei die Kanäle Kügelchen umfassen, die in einem Lösemittel löslich sind, das aus der Gruppe ausgewählt ist, die aus Wasser, Ethanol, Ether und Mischungen daraus besteht.

7. Transdermale Dosierungsform, umfassend:
eine Wirkstoffkomponente, die ein Polymermaterial und einen Wirkstoff umfasst, der in dem Polymermaterial dispergiert ist, wobei die Wirkstoffkomponente eine die Haut berührende Oberfläche aufweist,
eine Gegenwirkstoffkomponente, die einen Antagonisten zum Wirkstoff umfasst, und
eine diskontinuierliche Barriere, die für die Diffusion von Wirkstoff und Gegenwirkstoff undurchlässig ist, wobei die Barriere zwischen der Wirkstoffkomponente und der Gegenwirkstoffkomponente eingefügt ist,
wobei die Dosierungsform mindestens einen Kanal umfasst, der zwischen der die Haut berührenden Oberfläche und der Gegenwirkstoffkomponente verläuft.

8. Transdermale Dosierungsform nach Anspruch 1 oder 7, wobei der Kanal einen Luftkanal umfasst.

9. Transdermale Dosierungsform nach Anspruch 1 oder 7, ferner einen Träger umfassend, wobei die Gegenwirkstoffkomponente zwischen der Barriere und dem Träger eingefügt ist.

10. Transdermale Dosierungsform nach Anspruch 1 oder 7, wobei der Antagonist einen Antagonisten eines Narkotikums umfasst.

11. Transdermale Dosierungsform nach Anspruch 1 oder 7, wobei die Gegenmittelkomponente einen Haftklebstoff umfasst.

12. Transdermale Dosierungsform nach Anspruch 1 oder 7, wobei der Wirkstoff Fentanyl ist.

13. Transdermale Dosierungsform nach Anspruch 7, wobei die Barriere einen Film umfasst, der Öffnungen umfasst.

14. Transdermale Dosierungsform nach Anspruch 13, wobei die Öffnungen in der Barriere an Öffnungen in der Wirkstoffkomponente ausgerichtet sind.

15. Transdermale Dosierungsform nach Anspruch 14, wobei die Gegenwirkstoffkomponente Öffnungen umfasst, die an den Öffnungen der Barriere ausgerichtet sind.

16. Transdermale Dosierungsform nach Anspruch 7, wobei die Gegenwirkstoffkomponente einen porösen Film umfasst.

## Revendications

1. Forme d'administration transcutanée, qui comprend :
un composant d'agent actif qui comprend un agent actif dispersé dans un matériau polymère, le composant d'agent actif présentant une surface proximale de contact avec la peau et une surface distale, et au moins un canal qui assure la liaison entre la surface proximale et la surface distale,
un composant d'agent antagoniste qui comprend un antagoniste de l'agent actif et
une barrière interposée entre la surface distale du composant d'agent actif et le composant d'agent antagoniste,
la barrière étant perméable à un solvant sélectionné dans l'ensemble constitué de l'eau, de l'éthanol, de l'éther et de leurs mélanges, la barrière étant en outre imperméable à la diffusion de l'agent actif et de l'agent antagoniste en l'absence dudit solvant.

2. Forme d'administration transcutanée selon la revendication 1, dans laquelle la barrière est soluble dans un solvant sélectionné dans l'ensemble constitué de l'eau, de l'éthanol, de l'éther et de leurs mélanges.

3. Forme d'administration transcutanée selon la revendication 1, dans laquelle la barrière est perméable à l'eau, à l'éthanol et à leurs mélanges.

4. Forme d'administration transcutanée selon la revendication 1, dans laquelle la forme d'administration comprend plusieurs canaux qui assurent la liaison entre la surface proximale et la surface distale du composant d'agent actif.

5. Forme d'administration transcutanée selon la revendication 4, dans laquelle les canaux comprennent un matériau soluble dans un solvant sélectionné dans l'ensemble constitué de l'eau, de l'éthanol, de l'éther et de leurs mélanges.

6. Forme d'administration transcutanée selon la revendication 4, dans laquelle les canaux comprennent des billes solubles dans un solvant sélectionné dans l'ensemble constitué de l'eau, de l'éthanol, de l'éther et de leurs mélanges.

7. Forme d'administration transcutanée qui comprend :
un composant d'agent actif qui comprend un matériau polymère et un agent actif dispersé dans le matériau polymère, le composant d'agent actif ayant une surface en contact avec la peau,
un composant d'agent antagoniste qui contient un antagoniste de l'agent actif et
une barrière discontinue imperméable à la diffusion de l'agent actif et de l'agent antagoniste, la barrière étant intercalée entre le composant d'agent actif et le composant d'agent antagoniste,
la forme d'administration comprenant au moins un canal qui assure la liaison entre la surface en contact avec la peau et le composant d'agent antagoniste.

8. Forme d'administration transcutanée selon la revendication 1 ou 7, dans laquelle le canal comprend un canal d'air.

9. Forme d'administration transcutanée selon la revendication 1 ou 7, qui comprend en outre un dos, le composant d'agent antagoniste étant intercalé entre la barrière et le dos.

10. Forme d'administration transcutanée selon la revendication 1 ou 7, dans laquelle l'antagoniste contient un antagoniste narcotique.

11. Forme d'administration transcutanée selon la revendication 1 ou 7, dans laquelle le composant d'agent antagoniste comprend un adhésif sensible à la pression.

12. Forme d'administration transcutanée selon la revendication 1 ou 7, dans laquelle l'agent actif est le fentanyl.

13. Forme d'administration transcutanée selon la revendication 7, dans laquelle la barrière contient un film qui présente des ouvertures.

14. Forme d'administration transcutanée selon la revendication 13, dans laquelle les ouvertures ménagées dans la barrière sont alignées sur des ouvertures ménagées dans le composant d'agent actif.

15. Forme d'administration transcutanée selon la revendication 14, dans laquelle le composant d'agent antagoniste comprend des ouvertures alignées sur les ouvertures ménagées dans la barrière.

16. Forme d'administration transcutanée selon la revendication 7, dans laquelle le composant d'agent antagoniste comporte un film poreux.
